(19) Europäisches Patentamt
European Patent Office
Office européen des brevets

(11) EP 3 137 593 B1

(12) **FASCICULE DE BREVET EUROPEEN**

(45) Date de publication et mention de la délivrance du brevet:
**16.12.2020 Bulletin 2020/51**

(21) Numéro de dépôt: **15718945.7**

(22) Date de dépôt: **30.04.2015**

(51) Int Cl.:
***C12N 1/20*** *(2006.01)* ***C12P 7/62*** *(2006.01)*
*C12R 1/01* *(2006.01)*

(86) Numéro de dépôt international:
**PCT/EP2015/059609**

(87) Numéro de publication internationale:
**WO 2015/176940 (26.11.2015 Gazette 2015/47)**

(54) **NOUVELLE SOUCHE HYPERHALOPHILE ET SON UTILISATION POUR LA DEGRADATION DES SUBSTRATS CARBONES**

NEUER HYPERHALOPHILER STAMM UND DESSEN VERWENDUNG FÜR DEN ABBAU VON KOHLENSTOFFSUBSTRATEN

NOVEL HYPERHALOPHILIC STRAIN AND ITS USE FOR THE DEGRADATION OF CARBON SUBSTRATES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.05.2014 EP 14305655**

(43) Date de publication de la demande:
**08.03.2017 Bulletin 2017/10**

(73) Titulaires:
- **Institut De Recherche Pour Le Développement I.R.D.**
  **13572 Marseille Cedex 02 (FR)**
- **Société Interoleagineuse d' Assistance et de Développement (S.I.A.)**
  **75008 Paris (FR)**

(72) Inventeurs:
- **OLLIVIER, Bernard**
  **F-13360 Roquevaire (FR)**
- **BEN HANIA, Wajdi**
  **F-13009 Marseille (FR)**
- **THEVENIEAU, France**
  **F-28000 Chartres (FR)**
- **HEDI, Abdeljabbar**
  **2082 Tunis (TN)**
- **FARDEAU, Marie-Laure**
  **F-13170 Les Pennes Mirabeau (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**EP-A1- 2 202 316 WO-A2-2009/156950**

- **CARMEN HERMANN-KRAUSS ET AL: "Archaeal Production of Polyhydroxyalkanoate (PHA) Co- and Terpolyesters from Biodiesel Industry-Derived By-Products", ARCHAEA, vol. 50, no. 10, 1 janvier 2013 (2013-01-01), pages 1305-10, XP055131968, ISSN: 1472-3646, DOI: 10.1016/S1369-703X(03)00040-8**
- **SIMON-COLIN C ET AL: "Halomonas profundus sp. nov., a new PHA-producing bacterium isolated from a deep-sea hydrothermal vent shrimp", JOURNAL OF APPLIED MICROBIOLOGY, OXFORD, GB, vol. 104, 1 mai 2008 (2008-05-01), pages 1425-1432, XP008129838, ISSN: 1364-5072**
- **CHEN C W ET AL: "Enzymatic extruded starch as a carbon source for the production of poly(3-hydroxybutyrate-co-3-hydroxyvalerat e) by Haloferax mediterranei", PROCESS BIOCHEMISTRY, ELSEVIER, NL, vol. 41, no. 11, 1 novembre 2006 (2006-11-01), pages 2289-2296, XP027984007, ISSN: 1359-5113 [extrait le 2006-11-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

**(Cont. page suivante)**

- ARNAB PRAMANIK ET AL: "Utilization of vinasse for the production of polyhydroxybutyrate by Haloarcula marismortui", FOLIA MICROBIOLOGICA ; OFFICIAL JOURNAL OF THE INSTITUTE OF MICROBIOLOGY, ACADEMY OF SCIENCES OF THE CZECH REPUBLIC AND CZECHOSLAVAK SOCIETY FOR MICROBIOLOGY, SPRINGER NETHERLANDS, DORDRECHT, vol. 57, no. 1, 19 janvier 2012 (2012-01-19), pages 71-79, XP035026422, ISSN: 1874-9356, DOI: 10.1007/S12223-011-0092-3
- LOPEZ-CUELLAR M R ET AL: "Production of polyhydroxyalkanoates (PHAs) with canola oil as carbon source", INTERNATIONAL JOURNAL OF BIOLOGICAL MACROMOLECULES, ELSEVIER BV, NL, vol. 48, no. 1, 1 janvier 2011 (2011-01-01), pages 74-80, XP027564739, ISSN: 0141-8130 [extrait le 2010-12-17]
- AKHILESH KUMAR SINGH ET AL: "Exploitation of inexpensive substrates for production of a novel SCL-LCL-PHA co-polymer by Pseudomonas aeruginosa MTCC 7925", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 36, no. 3, 4 décembre 2008 (2008-12-04), pages 347-354, XP019665566, ISSN: 1476-5535
- Hans-Josef Endres ET AL: "Basics Basics of PHA", bioplastics MAGAZINE, 1 March 2011 (2011-03-01), pages 42-45, XP055514449, Retrieved from the Internet: URL:https://f2.hs-hannover.de/fileadmin/media/doc/ifbb/Bioplastics_Magazine__03_11__Vol._6_S._43-45.pdf [retrieved on 2018-10-11]
- Satoshi Tomizawa ET AL: "Molecular Weight Change of Polyhydroxyalkanoate (PHA) Caused by the PhaC Subunit of PHA Synthase from Bacillus cereus YB-4 in Recombinant Escherichia coli", Biomacromolecules, vol. 12, no. 7, 11 July 2011 (2011-07-11), pages 2660-2666, XP055036738, ISSN: 1525-7797, DOI: 10.1021/bm2004687

## Description

La présente invention a pour objet une nouvelle souche hyperhalophile et son utilisation pour la dégradation de substrats carbonés, permettant notamment la préparation de nouveaux polymères.

Chaque jour, des milliers de tonnes de déchets en plastique issu du pétrole s'accumulent dans l'environnement, entraînant un nombre croissant de sites d'enfouissement de déchets non biodégradables et l'escalade des coûts d'élimination des déchets.

Une solution à ce problème est d'utiliser des alternatives biodégradables à ces plastiques, l'une d'entre-elles étant la production de polyhydroxyalkanoates (PHA), une famille polymères biodégradables haute performance.

Les PHA peuvent être produits commercialement lors de processus de stockage intracellulaire (réserves) effectués par des micro-organismes utilisant un substrat approprié (notamment un sucre ou un acide organique). Cependant, le coût des PHA ainsi obtenus est bien supérieur à celui des plastiques issus du pétrole, du fait notamment des contrôles environnementaux stricts requis et des conditions opératoires stériles requises pour contrôler l'opération de production.

En outre, les PHA n'ont pas toujours les caractéristiques physiques adaptées à des applications industrielles spécifiques, en particulier la température de transition vitreuse (Tg) ou la transparence.

*Haloferax* est un genre d'*Archaea* appartenant à l'embranchement des *Euryarchaeota* et à l'ordre des *Halobacteriales.* Les cellules d'*Haloferax* sont des bâtonnets mobiles pléomorphes ou des disques aplatis, aérobies stricts. Ce sont des organismes hyperhalophiles.

Certaines espèces d'*Haloferax* produisent des biopolymères. En particulier, *Haloferax mediterranei* produit du PHA (Lillo et al. Appl. Environ. Microb. 56 :2517-2521, 1990).

Cependant, Lillo et al. décrit que *Haloferax mediterranei* produit seulement le PHA en conditions de stress, à partir d'amidon ou de glucose, et non à partir d'autres substrats carbonés tels que les huiles, les triglycérides, les acides gras ou le glycérol.

Hermann-Krauss et al. décrit la souche Haloferax mediterranei DSM 1411 qui est capable de produire du PHA d'un poids moléculaire de 150 à 253 kDa à partir du glycérol (Hermann-Krauss et al. Archae 50(10): 1305-1310, 2013).

Ainsi, un but de la présente invention est de disposer d'un nouveau microorganisme hyperhalophile capable de dégrader des substrats tels que les huiles, les triglycérides, les acides gras, les alcanes, des co-produits des raffineries d'huile, le glycérol, ou leurs dérivés, ledit microorganisme étant notamment non génétiquement modifié, et ayant la capacité de croître en milieu non stérile.

Un autre but de la présente invention consiste à disposer d'un nouveau microorganisme hyperhalophile capable de dégrader des substrats tels que les huiles, les triglycérides, les acides gras, les alcanes, des co-produits des raffineries d'huile, le glycérol, ou leurs dérivés, permettant notamment la préparation de PHA.

Un autre but de la présente invention consiste à disposer d'un nouveau microorganisme hyperhalophile capable de produire un PHA de haute masse moléculaire et/ou de haute transparence.

Un autre but de la présente invention consiste à disposer d'un nouveau microorganisme hyperhalophile capable de produire du PHA même dans des conditions normales de culture, c'est-à-dire sans stress.

L'invention a par conséquent pour objet l'utilisation de la souche hyperhalophile aérobie S3S1 déposée le 21 février 2014 à la CNCM sous le numéro CNCM I-4838,
pour la mise en œuvre d'un procédé de dégradation :

- d'un substrat constitué de ou comprenant un produit ou un co-produit de raffinerie d'oléagineux ou d'hydrocarbures liquides,
- et éventuellement d'un co-substrat carboné,

contenus dans le milieu de culture de la dite souche hyperhalophile aérobie.

Egalement décrit est l'utilisation de la souche hyperhalophile aérobie S3S1 déposée le 21 février 2014 à la CNCM sous le numéro CNCM I-4838, ou d'une souche hyperhalophile aérobie étant telle que le pourcentage d'hybridation ADN-ADN entre cette souche hyperhalophile aérobie et la souche S3S1 est d'au moins 90%, en particulier d'au moins 92%, pour la mise en œuvre d'un procédé de dégradation d'un substrat constitué de ou comprenant un produit ou un co-produit de raffinerie d'oléagineux ou d'hydrocarbures liquides, en l'absence de co-substrat carboné,
contenus dans le milieu de culture de la dite souche hyperhalophile aérobie.

Par « produit ou co-produit de raffinerie d'oléagineux ou d'hydrocarbures liquides », on entend notamment les huiles, les triglycérides, les acides gras, le glycérol, les alcanes, les alcènes, les polyènes, leurs dérivés ainsi que les compositions les comprenant»

Par « hydrocarbures liquides », on entend les hydrocarbures liquides à température ambiante et sous pression atmosphérique.

Par le pourcentage d'hybridation ADN-ADN, on entend le pourcentage de similitude entre deux génomes de

microorganismes.

L'hybridation ADN/ADN a été réalisée entre la souche S3S1 et la souche *Haloferax mediterranei,* selon les conditions ci-après.

Le pourcentage d'hybridation ADN-ADN est par exemple déterminé par la collection internationale de micro-organismes allemande (DSMZ) selon la méthode décrite par De Ley *et al.* (De Ley, J., Cattoir, H. & Reynaerts, A. (1970). The quantitative measurement of DNA hybridization from renaturation rates. Eur JBiochem 12, 133-142) et modifiée par Huss *et al.* (Huss, V. A. R., Festl, H. & Schleifer, K. H. (1983). Studies on the spectrophotometric détermination of DNA hybridization from renaturation rates. Syst Appl Microbiol 4, 184-192).

En particulier, le pourcentage d'hybridation ADN-ADN est déterminé en utilisant un spectrophotomètre UV/VIS (modèle Cary 100 Bio) équipé d'un changeur multicellulaire Peltier 6x6 thermostaté et un contrôleur de température avec une sonde de température in-situ (Varian).

L'invention a également pour objet l'utilisation de la souche hyperhalophile aérobie S3S1 déposée le 21 février 2014 à la CNCM sous le numéro CNCM I-4838, pour la mise en œuvre d'un procédé de dégradation :

- d'un substrat constitué de ou comprenant un élément choisi parmi les triglycérides, leurs sous-produits, les alcanes, les alcènes, les polyènes et leurs mélanges ;
- et éventuellement d'un co-substrat carboné,

contenus dans le milieu de culture de la dite souche hyperhalophile aérobie.

La description a également pour objet l'utilisation de la souche hyperhalophile aérobie S3S1 déposée le 21 février 2014 à la CNCM sous le numéro CNCM I-4838, ou d'une souche hyperhalophile aérobie étant telle que le pourcentage d'hybridation ADN-ADN entre cette souche hyperhalophile aérobie et la souche S3S1 est d'au moins 90%, en particulier d'au moins 92%, pour la mise en œuvre d'un procédé de dégradation :

d'un substrat constitué de ou comprenant un élément choisi parmi les triglycérides, leurs sous-produits, les alcanes, les alcènes, les polyènes et leurs mélanges, en l'absence de co-substrat carboné,
contenus dans le milieu de culture de la dite souche hyperhalophile aérobie.

L'invention a également pour objet l'utilisation de la souche hyperhalophile aérobie S3S1 déposée le 21 février 2014 à la CNCM sous le numéro CNCM I-4838, ,
pour la mise en œuvre d'un procédé de dégradation :

- d'un substrat constitué de ou comprenant un produit ou un co-produit de raffinerie d'oléagineux ou d'hydrocarbures liquides, ledit produit ou co-produit comprenant :

    o au moins 20% en masse d'un élément choisi parmi les matières grasses, les triglycérides, le glycérol, les acides gras, leurs dérivés et leurs mélanges ; ou
    o au moins 20% en masse d'un élément choisi parmi les alcanes, les alcènes, les polyènes, et leurs mélanges, lesdits alcanes, alcènes et polyènes comprenant de 6 à 24 atomes de carbone, en particulier de 10 à 22 atomes de carbone ;

- et éventuellement d'un co-substrat carboné,

contenus dans le milieu de culture de la dite souche hyperhalophile aérobie.

L'invention a également pour objet l'utilisation de la souche hyperhalophile aérobie S3S1 déposée le 21 février 2014 à la CNCM sous le numéro CNCM I-4838, pour la mise en œuvre d'un procédé de dégradation :

- d'un substrat constitué de ou comprenant un produit ou un co-produit de raffinerie d'oléagineux ou d'hydrocarbures liquides, ledit produit ou co-produit comprenant :

    ◦ au moins 20% en masse d'un élément choisi parmi les matières grasses, les triglycérides, le glycérol, les acides gras, leurs dérivés et leurs mélanges ; ou
    ◦ au moins 20% en masse d'un élément choisi parmi les alcanes, les alcènes, les polyènes, et leurs mélanges, lesdits alcanes, alcènes et polyènes comprenant de 6 à 24 atomes de carbone, en particulier de 10 à 22 atomes de carbone, en l'absence de co-substrat carboné,

contenus dans le milieu de culture de la dite souche hyperhalophile aérobie.

Par « matières grasses », on entend notamment les huiles, les graisses, les lipides.

Les Inventeurs ont mis en évidence l'existence d'une nouvelle souche hyperhalophile aérobie d'*Haloferax* qui, même dans des conditions normales de culture, c'est-à-dire sans stress, et aérobies, dégrade des substrats de type huile, triglycérides, acides gras ou leurs dérivés, glycérol, alcanes, alcènes, polyènes, et éventuellement des co-substrats carbonés.

Les Inventeurs ont également mis en évidence l'existence d'une nouvelle souche hyperhalophile aérobie d'*Haloferax* qui, même dans des conditions normales de culture, c'est-à-dire sans stress, et aérobies, dégrade des substrats de type huile, triglycérides, acides gras ou leurs dérivés, glycérol, alcanes, alcènes, polyènes, en l'absence de co-substrat carboné.

L'expression « en l'absence de co-substrat carboné », signifie que le milieu de culture est exclusivement hydrophobe.

Par « souche hyperhalophile », on entend une souche qui nécessite de fortes concentrations en sels, en particulier NaCl, $MgCl_2$ et/ou $CaCl_2$, dans son milieu de culture pour croître. Il s'agit en particulier d'une souche se développant, notamment de manière optimale, dans un milieu de culture dont la concentration en sel, en particulier NaCl, est compris d'environ 100 g/L à environ 300 g/L, notamment d'environ 100 g/L à environ 250 g/L.

Lorsque le sel est en fait un mélange de sels, la concentration comprise d'environ 100 g/L à environ 300 g/L, notamment d'environ 100 g/L à environ 250 g/L correspond à la concentration totale en sels dudit mélange.

Ledit milieu de culture comprend en particulier NaCl, et comprend éventuellement en outre $MgCl_2$ et/ou $CaCl_2$ à des concentrations comprises d'environ 2g/L à environ 20 g/L.

Par « dégradation », on entend la transformation du dit substrat et éventuellement du dit co-substrat carboné, par la dite souche, en un ou plusieurs composés d'intérêt.

Les composés d'intérêt sont en particulier les biopolymères de type polyhydroxyalkanoate (PHA), la bactériorhodopsine et les estérases halostables.

Par « sous-produits des triglycérides », on entend notamment le glycérol, les acides gras, les dérivés d'acides gras, en particulier les esters méthyliques et éthyliques des dits acides gras, ou les co-produits des raffineries d'huile.

Les co-produits des raffineries d'huile sont notamment des compositions comprenant du glycérol, des acides gras, des dérivés d'acides gras, en particulier les esters méthyliques et éthyliques des dits acides gras, ou leurs mélanges.

En particulier, les substrats comprenant des triglycérides sont notamment des huiles végétales ou animales et leurs dérivés, en particulier les huiles de friture.

Le substrat est notamment choisi parmi le groupe constitué :

- des huiles végétales ou animales, en particulier les huiles végétales, plus particulièrement l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, leurs mélanges, et leurs dérivés, en particulier les huiles de friture ;
- des alcanes linéaires, ramifiés ou cycliques, comprenant de 6 à 24 atomes de carbone, en particulier de 10 à 22 atomes de carbone ;
- des alcènes linéaires, ramifiés ou cycliques, comprenant de 6 à 24 atomes de carbone, en particulier de 10 à 22 atomes de carbone ;
- des polyènes linéaires, ramifiés ou cycliques, comprenant de 6 à 24 atomes de carbone, en particulier de 10 à 22 atomes de carbone ;
- des acides gras, en particulier les acides gras saturés et insaturés, plus particulièrement les acides gras mono insaturés, di insaturés et poly insaturés, lesdits acides gras comprenant un acide carboxylique et une chaîne comprenant de 6 à 24 atomes de carbone, en particulier de 10 à 22 atomes de carbone, ainsi que leurs dérivés, en particulier les esters des dits acides gras, plus particulièrement les esters méthyliques et éthyliques des dits acides gras;
- des trigycérides ;
- du glycérol ;
- des co-produits des raffineries d'huile, en particulier d'huile végétale, notamment les pâtes de neutralisation, les condensats de désodorisation, les gommes, les distillats d'acides gras, les graisses d'aéroflottateurs et les boues de station d'épuration.

Par polyène, on entend un composé comprenant au moins deux doubles liaisons carbone-carbone.

Par « pâtes de neutralisation », on entend le co-produit notamment obtenu par neutralisation desdites huiles à la soude.

Par « condensats de désodorisation », on entend notamment le co-produit obtenu lors du lavage des vapeurs issues de la désodorisation desdites huiles.

Par « gommes », on entend notamment le co-produit obtenu à l'issue de la centrifugation desdites huiles lors d'un dégommage acide.

Par «distillats d'acides gras », on entend notamment le co-produit obtenu lors de d'une distillation neutralisante desdites huiles.

Par « graisses d'aéroflottateurs », on entend notamment le co-produit obtenu par traitement de type aéroflottateur d'effluents aqueux d'usines.

Par « boues de station d'épuration », on entend notamment le sous-produit obtenu par traitement des effluents liquides d'usines.

Par « co-substrat carboné », on entend un composé comprenant au moins un atome de carbone, ledit composé étant susceptible d'être dégradé par la dite souche.

Le substrat carboné est notamment un sucre, en particulier le glucose.

Par « milieu de culture de la dite souche hyperhalophile», on entend un milieu de culture adapté à la dite souche hyperhalophile, c'est-à-dire un milieu de culture contenant les éléments nutritifs, organiques et minéraux, nécessaires à la croissance de la dite souche hyperhalophile. Il s'agit notamment d'un milieu de culture qui comprend, outre le dit substrat et éventuellement le co-substrat carboné, des sels, en particulier NaCl, à une concentration comprise d'environ 100 g/L à environ 300 g/L, notamment d'environ 100 g/L à environ 250 g/L, ledit milieu de culture comprenant éventuellement en outre $MgCl_2$ et/ou $CaCl_2$ à des concentrations comprises d'environ 2g/L à environ 20 g/L.

Le milieu de culture peut en particulier comprendre une source d'azote, par exemple une source d'azote inorganique, y compris le sulfate d'ammonium, le chlorure d'ammonium ou le nitrate d'ammonium, ou une source d'azote organique, en particulier un extrait de levure, la peptone ou un extrait de viande. En plus de ceux-ci, le dit milieu peut en outre contenir des minéraux, des sels métalliques, et /ou des vitamines, si nécessaire.

Selon un mode de réalisation avantageux, l'invention concerne une utilisation telle que définie précédemment, dans laquelle ledit substrat est, dans ledit milieu de culture, la seule source de carbone et/ou d'énergie de ladite souche hyperhalophile aérobie.

Selon un mode de réalisation avantageux, l'invention concerne une utilisation telle que définie précédemment, dans laquelle ledit substrat est, dans ledit milieu de culture, la principale source de carbone et/ou d'énergie de ladite souche hyperhalophile aérobie.

Par « principale source de carbone et/ou d'énergie », on entend une source de carbone et/ou d'énergie correspondant à plus de 50%, en particulier plus de 60%, 70%, 80% ou 90%, en masse du total des sources de carbone et/ou d'énergie de ladite souche hyperhalophile aérobie.

Une source de carbone et/ou d'énergie secondaire peut être des levures, lorsque ces dernières sont présentes dans le milieu de culture.

Selon un mode de réalisation avantageux, la description concerne une utilisation telle que définie précédemment, d'une souche hyperhalophile aérobie dont le génome total présente un pourcentage d'identité d'au moins 90%, 91%, 92%, 93%, 94%, 95%, 96%, 97%, 98% ou 99% avec le génome total de la dite souche S3S1.

Egalement décrit est une utilisation telle que définie précédemment, d'une souche hyperhalophile aérobie étant telle que le pourcentage d'hybridation ADN-ADN entre cette souche hyperhalophile aérobie et la souche S3S1 est d'au moins 90%, 91%, 92%, 93%, 94%, 95%, 96%, 97%, 98% ou 99%.

Selon un autre aspect, l'invention concerne un procédé de dégradation :

- un substrat constitué de ou comprenant un produit ou un co-produit de raffinerie d'oléagineux ou d'hydrocarbures liquides;
- et éventuellement un co-substrat carboné;

comprenant une étape de croissance d'une souche hyperhalophile aérobie S3S1 déposée le 21 février 2014 à la CNCM sous le numéro CNCM I-4838, dans un milieu de culture comprenant :

- un substrat constitué de ou comprenant un élément choisi parmi les triglycérides, leurs sous-produits, les alcanes, les alcènes, les polyènes et leurs mélanges ;
- et éventuellement un co-substrat carboné.

Selon un autre aspect, l'invention concerne un procédé de dégradation :

- un substrat constitué de ou comprenant un élément choisi parmi les triglycérides, leurs sous-produits, les alcanes, les alcènes, les polyènes et leurs mélanges ;
- et éventuellement un co-substrat carboné ;

comprenant une étape de croissance d'une souche hyperhalophile aérobie S3S1 déposée le 21 février 2014 à la CNCM sous le numéro CNCM I-4838, dans un milieu de culture comprenant :

- un substrat constitué de ou comprenant un élément choisi parmi les triglycérides, leurs sous-produits, les alcanes, les alcènes, les polyènes et leurs mélanges ;
- et éventuellement un co-substrat carboné.

Selon un autre aspect, l'invention concerne un procédé de dégradation :

- un substrat constitué de ou comprenant un produit ou un co-produit de raffinerie d'oléagineux ou d'hydrocarbures liquides, en l'absence de co-substrat carboné;

comprenant une étape de croissance d'une souche hyperhalophile aérobie S3S1 déposée le 21 février 2014 à la CNCM sous le numéro CNCM I-4838, dans un milieu de culture comprenant :

- un substrat constitué de ou comprenant un élément choisi parmi les triglycérides, leurs sous-produits, les alcanes, les alcènes, les polyènes et leurs mélanges, en l'absence de co-substrat carboné.

Selon un autre aspect, l'invention concerne un procédé de dégradation :

- un substrat constitué de ou comprenant un élément choisi parmi les triglycérides, leurs sous-produits, les alcanes, les alcènes, les polyènes et leurs mélanges, en l'absence de co-substrat carboné ;

comprenant une étape de croissance d'une souche hyperhalophile aérobie S3S1 déposée le 21 février 2014 à la CNCM sous le numéro CNCM I-4838, dans un milieu de culture comprenant :

- un substrat constitué de ou comprenant un élément choisi parmi les triglycérides, leurs sous-produits, les alcanes, les alcènes, les polyènes et leurs mélanges, en l'absence de co-substrat carboné.

Par « étape de croissance », on entend une étape dans laquelle la biomasse de la dite souche augmente, et dans laquelle du substrat et éventuellement du co-substrat carboné, sont consommés.

Ainsi, la dite étape de croissance consiste en une mise en contact de ladite souche hyperhalophile aérobie S3S1 dans son milieu de culture initial (c'est-à-dire un milieu de culture sans le dit substrat et le cas échéant sans le dit co-substrat carboné) avec ledit substrat et éventuellement le dit co-substrat carboné, formant le « milieu de culture de la dite souche hyperhalophile » tel que défini précédemment.

A l'issue de ladite étape de croissance de ladite souche, du substrat et éventuellement du co-substrat carboné sont consommés et transformés en un ou plusieurs composés d'intérêt.

Selon un mode de réalisation avantageux, la description concerne un procédé tel que défini précédemment, comprenant une étape de croissance d'une souche hyperhalophile aérobie dont le génome total présente un pourcentage d'identité d'au moins 90%, 91%, 92%, 93%, 94%, 95%, 96%, 97%, 98% ou 99% avec le génome total de la dite souche S3S1.

Selon un mode de réalisation avantageux, la description concerne un procédé tel que défini précédemment, comprenant une étape de croissance d'une souche hyperhalophile aérobie étant telle que le pourcentage d'hybridation ADN-ADN entre cette souche hyperhalophile aérobie et la souche S3S1 est d'au moins 90%, 91%, 92%, 93%, 94%, 95%, 96%, 97%, 98% ou 99%.

Selon un mode de réalisation avantageux, l'invention concerne un procédé tel que défini précédemment, dans lequel le milieu de culture de ladite souche comprend un substrat, et non un co-substrat carboné.

Selon un mode de réalisation avantageux, l'invention concerne un procédé tel que défini précédemment, dans lequel le milieu de culture de ladite souche comprend un substrat choisi parmi le groupe constitué :

- des huiles végétales et animales, en particulier les huiles végétales, plus particulièrement l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, leurs mélanges, et leurs dérivés, en particulier les huiles de friture ;
- des alcanes linéaires, ramifiés ou cycliques, comprenant de 6 à 24 atomes de carbone, en particulier de 10 à 22 atomes de carbone ;
- des alcènes linéaires, ramifiés ou cycliques, comprenant de 6 à 24 atomes de carbone, en particulier de 10 à 22 atomes de carbone ;
- des polyènes linéaires, ramifiés ou cycliques, comprenant de 6 à 24 atomes de carbone, en particulier de 10 à 22 atomes de carbone ;
- des acides gras, en particulier les acides gras saturés et insaturés, plus particulièrement les acides gras mono

insaturés, di insaturés et poly insaturés, lesdits acides gras comprenant un acide carboxylique et une chaîne comprenant de 6 à 24 atomes de carbone, en particulier de 10 à 22 atomes de carbone ainsi que leurs dérivés, en particulier les esters des dits acides gras, plus particulièrement les esters méthyliques et éthyliques des dits acides gras;
- des trigycérides ;
- du glycérol ;
- des co-produits des raffineries d'huile, en particulier d'huile végétale, notamment les pâtes de neutralisation, les condensats de désodorisation, les gommes, les distillats d'acides gras, les graisses d'aéroflottateurs et les boues de station d'épuration,

et leurs mélanges,
contenu dans le milieu de culture de la dite souche hyperhalophile aérobie.

Selon un mode de réalisation avantageux, l'invention concerne un procédé tel que défini précédemment, dans lequel ledit substrat est :

- une huile végétale, en particulier l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, plus particulièrement l'huile de colza ou l'huile de tournesol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- le glycérol,

ou l'un de leurs mélanges.

Selon un mode de réalisation avantageux, l'invention concerne un procédé tel que défini précédemment, dans lequel le milieu de culture de ladite souche comprend du glycérol.

Selon un mode de réalisation avantageux, l'invention concerne un procédé tel que défini précédemment, dans lequel le milieu de culture de ladite souche comprend un substrat et un co-substrat carboné.

Selon un mode de réalisation avantageux, l'invention concerne un procédé tel que défini précédemment, dans lequel le milieu de culture de ladite souche comprend du glycérol et un co-substrat carboné.

Selon un mode de réalisation avantageux, l'invention concerne un procédé tel que défini précédemment, dans lequel le milieu de culture de ladite souche comprend un substrat et un co-substrat carboné, ledit co-substrat étant un sucre, en particulier le glucose.

Selon un mode de réalisation avantageux, l'invention concerne un procédé tel que défini précédemment, dans lequel le milieu de culture de ladite souche comprend un substrat et du glucose.

Selon un mode de réalisation avantageux, l'invention concerne un procédé tel que défini précédemment, dans lequel le milieu de culture de ladite souche comprend un substrat et un co-substrat carboné, ledit co-substrat étant en particulier un sucre, notamment le glucose, la concentration en co-substrat étant comprise de 1g à 100g/L, notamment de 1g à 20g/L, en particulier de 1g à 10g/L, plus particulièrement de 1g à 5g/L.

Selon un mode de réalisation avantageux, l'invention concerne un procédé tel que défini précédemment, dans lequel le milieu de culture de ladite souche comprend un substrat en l'absence de co-substrat carboné.

Selon un mode de réalisation avantageux, l'invention concerne un procédé tel que défini précédemment, dans lequel le milieu de culture de ladite souche comprend :

❖ un substrat choisi parmi le groupe constitué :

  - des huiles végétales ou animales, en particulier les huiles végétales, plus particulièrement l'huile de colza, l'huile de tournesol, l'huile de maïs, l'huile de lin, l'huile d'olive, l'huile de ricin, l'huile de soja, l'huile de palme,

l'huile palmiste, l'huile de coco, l'huile de graine de coton, l'huile d'arachide, leurs mélanges, et leurs dérivés, en particulier les huiles de friture;
- des alcanes linéaires, ramifiés ou cycliques, comprenant de 6 à 24 atomes de carbone, en particulier de 10 à 22 atomes de carbone ;
- des alcènes linéaires, ramifiés ou cycliques, comprenant de 6 à 24 atomes de carbone, en particulier de 10 à 22 atomes de carbone ;
- des polyènes linéaires, ramifiés ou cycliques, comprenant de 6 à 24 atomes de carbone, en particulier de 10 à 22 atomes de carbone ;
- des acides gras, en particulier les acides gras saturés et insaturés, plus particulièrement les acides gras mono insaturés, di insaturés et poly insaturés, lesdits acides gras comprenant un acide carboxylique et une chaîne comprenant de 6 à 24 atomes de carbone, en particulier de 10 à 22 atomes de carbone, ainsi que leurs dérivés, en particulier les esters des dits acides gras, plus particulièrement les esters méthyliques et éthyliques des dits acides gras;
- des trigycérides ;
- du glycérol ;
- des co-produits des raffineries d'huile, en particulier d'huile végétale, notamment les pâtes de neutralisation, les condensats de désodorisation, les gommes, les distillats d'acides gras, les graisses d'aéroflottateurs et les boues de station d'épuration,

et leurs mélanges,

❖ un co-substrat carboné, ledit co-substrat étant un sucre, en particulier le glucose, contenus dans le milieu de culture de la dite souche hyperhalophile aérobie.

Selon un mode de réalisation avantageux, l'invention concerne un procédé tel que défini précédemment, dans lequel le milieu de culture de ladite souche comprend :

❖ un substrat, ledit substrat étant:

- une huile végétale, en particulier l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, plus particulièrement l'huile de colza ou l'huile de tournesol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- le glycérol,

ou l'un de leurs mélanges,

❖ un co-substrat carboné, ledit co-substrat étant un sucre, en particulier le glucose, contenus dans le milieu de culture de la dite souche hyperhalophile aérobie.

Selon un mode de réalisation avantageux, l'invention concerne un procédé tel que défini précédemment, dans lequel le milieu de culture de ladite souche comprend du glycérol, et un co-substrat carboné, ledit co-substrat étant un sucre, en particulier le glucose.

Selon un mode de réalisation avantageux, l'invention concerne un procédé tel que défini précédemment, dans lequel le milieu de culture de ladite souche comprend un substrat et éventuellement un co-substrat carboné choisis parmi du glycérol et du stéarate, du glycérol et du stéarate de méthyle, du glycérol et de l'acide oléique, du glycérol et de l'oléate de méthyle, du glycérol et de l'acide érucique, du glycérol et de l'érucate de méthyle, du glycérol et de l'acide laurique, du glycérol et du laurate de méthyle, du glycérol et de l'huile, du glycérol et de l'huile de colza, du glycérol et

de l'huile de tournesol, du glucose et du stéarate, du glucose et du stéarate de méthyle, du glucose et de l'acide oléique, du glucose et de l'oléate de méthyle, du glucose et de l'acide érucique, du glucose et de l'érucate de méthyle, du glucose et de l'acide laurique, du glucose et du laurate de méthyle, du glucose et de l'huile, du glucose et de l'huile de colza, ou du glucose et de l'huile de tournesol.

Selon un mode de réalisation avantageux, l'invention concerne un procédé tel que défini précédemment, dans lequel le milieu de culture comprend au moins un sel.

Par sel, on entend notamment un sel dont s'accommode ou a besoin la dite souche hyperhalophile.

Selon un mode de réalisation avantageux, l'invention concerne un procédé tel que défini précédemment, dans lequel le sel est choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment, dans lequel ledit sel est présent dans le milieu de culture à une concentration comprise d'environ 100 g/L à environ 300 g/L, notamment d'environ 100 g/L à environ 250 g/L.

Lorsque le sel est en fait un mélange de sels, la concentration comprise d'environ 100 g/L à environ 300 g/L, notamment d'environ 100 g/L à environ 250 g/L correspond à la concentration totale en sels dudit mélange.

En particulier, ladite souche hyperhalophile se développe de manière optimale dans un milieu de culture comprenant d'environ 100 g/L à environ 250 g/L de sel(s).

Une telle concentration en sel(s) permet d'éviter toute contamination du milieu extérieur en l'absence de stérilisation du milieu de culture.

Selon un mode de réalisation avantageux, l'invention concerne un procédé tel que défini précédemment, dans lequel le milieu de culture de ladite souche comprend :

❖ un substrat choisi parmi :

- l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, en particulier l'huile de colza ou l'huile de tournesol,
- le glycérol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,

et leurs mélanges,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment, dans lequel le milieu de culture de ladite souche comprend :

❖ du glycérol,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment, dans lequel le milieu de culture de ladite souche comprend :

❖ un substrat choisi parmi :

- l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, en particulier l'huile de colza ou l'huile de tournesol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,

et leurs mélanges,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment, dans lequel le milieu de culture de ladite souche comprend :

❖ de l'huile de colza, en tant que substrat,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant en particulier un mélange comprenant NaCl, KCl, $CaCl_2.2H_2O$ et $MgCl_2.6H_2O$.

Selon un mode de réalisation avantageux, l'invention concerne un procédé tel que défini précédemment, dans lequel le milieu de culture de ladite souche comprend :

❖ de l'acide oléique, en tant que substrat,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant en particulier un mélange comprenant NaCl, KCl, $CaCl_2.2H_2O$ et $MgCl_2.6H_2O$.

Selon un mode de réalisation avantageux, l'invention concerne un procédé tel que défini précédemment, dans lequel le milieu de culture de ladite souche comprend :
❖ du glycérol, en tant que substrat,
et
un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant en particulier un mélange comprenant NaCl, KCl, $CaCl_2.2H_2O$ et $MgCl_2.6H_2O$.
Selon un mode de réalisation avantageux, l'invention concerne un procédé tel que défini précédemment, dans lequel le milieu de culture de ladite souche comprend :

❖ un substrat choisi parmi :

- l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, en particulier l'huile de colza ou l'huile de tournesol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,

- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- le glycérol,

et leurs mélanges,

❖ un co-substrat carboné, en particulier un sucre, notamment le glucose,

et

❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment, dans lequel le milieu de culture de ladite souche comprend :

❖ du glycérol, en tant que substrat,

❖ un co-substrat carboné, en particulier un sucre, notamment le glucose,

et

❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment, dans lequel le milieu de culture de ladite souche comprend :

❖ de l'huile de colza, en tant que substrat,

❖ du glucose, en tant que co-substrat,

et

❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant en particulier un mélange comprenant NaCl, KCl, $CaCl_2.2H_2O$ et $MgCl_2.6H_2O$.

Selon un mode de réalisation avantageux, l'invention concerne un procédé tel que défini précédemment, dans lequel le milieu de culture de ladite souche comprend :

❖ de l'acide oléique, en tant que substrat,

❖ du glucose, en tant que co-substrat,

et

❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant en particulier un mélange comprenant NaCl, KCl, $CaCl_2.2H_2O$ et $MgCl_2.6H_2O$.

Selon un mode de réalisation avantageux, l'invention concerne un procédé tel que défini précédemment, dans lequel le milieu de culture de ladite souche comprend :

❖ du glycérol, en tant que substrat,

❖ du glucose, en tant que co-substrat,

et

❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant en particulier un mélange comprenant NaCl, KCl, $CaCl_2.2H_2O$ et $MgCl_2.6H_2O$.

Selon un mode de réalisation avantageux, l'invention concerne un procédé tel que défini précédemment, dans lequel le milieu de culture de ladite souche comprend :

❖ un substrat choisi parmi :

- l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, en particulier l'huile de colza ou l'huile de tournesol,
- le glycérol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, α-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, α-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,

et leurs mélanges,
ledit substrat étant présent dans le milieu de culture à une concentration comprise de 1 à 100 g/l,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges,

ledit sel étant présent dans le milieu de culture à une concentration comprise d'environ 100 g/L à environ 300 g/L, notamment d'environ 100 g/L à environ 250 g/L.

Selon un mode de réalisation avantageux, l'invention concerne un procédé tel que défini précédemment, dans lequel le milieu de culture de ladite souche comprend :

❖ du glycérol,
ledit glycérol étant présent dans le milieu de culture à une concentration comprise de 1 à 100 g/l,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges,

ledit sel étant présent dans le milieu de culture à une concentration comprise d'environ 100 g/L à environ 300 g/L, notamment d'environ 100 g/L à environ 250 g/L.

Selon un mode de réalisation avantageux, l'invention concerne un procédé tel que défini précédemment, dans lequel le milieu de culture de ladite souche comprend :

❖ un substrat choisi parmi :

- l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, en particulier l'huile de colza ou l'huile de tournesol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, α-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, α-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,

- le glycérol,

et leurs mélanges,
ledit substrat étant présent dans le milieu de culture à une concentration comprise de 1 à 100 g/l,
❖ un co-substrat carboné, ledit co-substrat étant en particulier un sucre, notamment le glucose, la concentration en co-substrat étant comprise de 1g à 100g/L, notamment de 1g à 20g/L, en particulier de 1g à 10g/L, plus particulièrement de 1g à 5g/L,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges,

ledit sel étant présent dans le milieu de culture à une concentration comprise d'environ 100 g/L à environ 300 g/L, notamment d'environ 100 g/L à environ 250 g/L.

Selon un mode de réalisation avantageux, l'invention concerne un procédé tel que défini précédemment, dans lequel le milieu de culture de ladite souche comprend :

❖ du glycérol,
ledit glycérol étant présent dans le milieu de culture à une concentration comprise de 1 à 100 g/l,
❖ un co-substrat carboné, ledit co-substrat étant en particulier un sucre, notamment le glucose, la concentration en co-substrat étant comprise de 1g à 100g/L, notamment de 1g à 20g/L, en particulier de 1g à 10g/L, plus particulièrement de 1g à 5g/L,
et
❖ un sel choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges,

ledit sel étant présent dans le milieu de culture à une concentration comprise d'environ 100 g/L à environ 300 g/L, notamment d'environ 100 g/L à environ 250 g/L.

Selon un mode de réalisation avantageux, l'invention concerne un procédé tel que défini précédemment, dans lequel le milieu de culture de ladite souche comprend :

- de l'huile de colza, de l'acide oléique ou du glycérol, à une concentration comprise de 1 à 100 g/l,
- NaCl, à une concentration comprise d'environ 100 g/L à environ 300 g/L, notamment d'environ 100 g/L à environ 250 g/L,
- KCl, à une concentration comprise de 0 à 20 g/L, en particulier de 2 g/L à 20 g/L,
- $CaCl_2.2H_2O$, à une concentration comprise de 0 à 20 g/L, en particulier de 2 g/L à 20 g/L,
- $MgCl_2.6H_2O$, à une concentration comprise de 0 à 20 g/L, en particulier de 2 g/L à 20 g/L.

Selon un mode de réalisation avantageux, l'invention concerne un procédé tel que défini précédemment, dans lequel le milieu de culture de ladite souche comprend :

- de l'huile de colza, de l'acide oléique ou du glycérol, à une concentration comprise de 1 à 100 g/l,
- du glucose, à une concentration comprise de 1g à 100g/L, notamment de 1g à 20g/L, en particulier de 1g à 10g/L, plus particulièrement de 1g à 5g/L,
- NaCl, à une concentration comprise d'environ 100 g/L à environ 300 g/L, notamment d'environ 100 g/L à environ 250 g/L,
- KCl, à une concentration comprise de 0 à 20 g/L, en particulier de 2 g/L à 20 g/L,
- $CaCl_2.2H_2O$, à une concentration comprise de 0 à 20 g/L, en particulier de 2 g/L à 20 g/L,
- $MgCl_2.6H_2O$, à une concentration comprise de 0 à 20 g/L, en particulier de 2 g/L à 20 g/L.

Selon un mode de réalisation avantageux, l'invention concerne un procédé tel que défini précédemment dans lequel la dite souche est cultivée dans un milieu dans lequel le rapport massique $NaCl/MgCl_2$ est compris de 15 à 100.

Selon un mode de réalisation avantageux, l'invention concerne un procédé tel que défini précédemment dans lequel la dite souche est cultivée dans un milieu dans lequel le rapport massique $NaCl/CaCl_2$ est compris de 20 à 100.

Lorsque, dans le milieu de culture, le rapport massique $NaCl/MgCl_2$ est compris de 15 à 100, et/ou le rapport massique $NaCl/CaCl_2$ est compris de 20 à 100, la croissance de la souche est améliorée par rapport à celle de la même souche dans un milieu dans lequel le rapport massique $NaCl/MgCl_2$ n'est pas compris de 15 à 100, et/ou dans lequel le rapport massique $NaCl/CaCl_2$ n'est pas compris de 20 à 100, en particulier un milieu ne comprenant pas de $MgCl_2$ et/ou de $CaCl_2$.

Selon un mode de réalisation avantageux, l'invention concerne un procédé tel que défini précédemment dans lequel la dite souche est cultivée sous une atmosphère comprenant moins de 21% en volume de dioxygène, en particulier moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène.

Selon un mode de réalisation avantageux, l'invention concerne un procédé tel que défini précédemment dans lequel le rapport carbone sur azote dans le milieu de culture est compris de 1 à 300.

Lorsque le rapport carbone sur azote dans le milieu de culture est compris de 1 à 300, la dite souche peut être en conditions de stress, et la quantité de substrat dégradé peut alors être augmentée.

Selon un mode de réalisation avantageux, l'invention concerne un procédé tel que défini précédemment dans lequel le rapport carbone sur phosphore dans le milieu de culture est compris de 80 à 800.

Lorsque le rapport carbone sur phosphore dans le milieu de culture est compris de 80 à 800, la dite souche peut être en conditions de stress, et la quantité de substrat dégradé peut alors être augmentée.

Selon un mode de réalisation avantageux, l'invention concerne un procédé tel que défini précédemment dans lequel la dite souche est cultivée dans un milieu dans lequel :

- le rapport massique $NaCl/MgCl_2$ est compris de 15 à 100, et/ou
- le rapport massique $NaCl/CaCl_2$ est compris de 20 à 100.

Selon un mode de réalisation avantageux, l'invention concerne un procédé tel que défini précédemment dans lequel :

- la dite souche est cultivée dans un milieu dans lequel le rapport massique $NaCl/MgCl_2$ est compris de 15 à 100, et/ou le rapport massique $NaCl/CaCl_2$ est compris de 20 à 100,
- la dite souche est cultivée sous une atmosphère comprenant moins de 21% en volume de dioxygène, en particulier moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène,
- le rapport carbone sur azote dans le milieu de culture est compris de 1 à 300, le rapport carbone sur phosphore dans le milieu de culture est compris de 80 à 800.

Selon un mode de réalisation avantageux, l'invention concerne un procédé tel que défini précédemment dans lequel le milieu de culture de ladite souche comprend :

◦ de l'huile de colza, de l'acide oléique ou du glycérol, à une concentration comprise de 1 à 100 g/l,
◦ éventuellement du glucose, à une concentration comprise de 1g à 100g/L, notamment de 1g à 20g/L, en particulier de 1g à 10g/L, plus particulièrement de 1g à 5g/L,
◦ NaCl, à une concentration comprise d'environ 100 g/L à environ 300 g/L, notamment d'environ 100 g/L à environ 250 g/L,
◦ KCl, à une concentration comprise de 0 à 20 g/L, en particulier de 2 g/L à 20 g/L,
◦ $CaCl_2.2H_2O$, à une concentration comprise de 0 à 20 g/L, en particulier de 2 g/L à 20 g/L,
◦ $MgCl_2.6H_2O$, à une concentration comprise de 0 à 20 g/L, en particulier de 2 g/L à 20 g/L,

le rapport massique $NaCl/MgCl_2$ étant compris de 15 à 100, et/ou le rapport massique $NaCl/CaCl_2$ étant compris de 20 à 100.

Selon un mode de réalisation avantageux, l'invention concerne un procédé tel que défini précédemment dans lequel :

- la dite souche est cultivée sous une atmosphère comprenant moins de 21% en volume de dioxygène, en particulier moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène,
- le rapport carbone sur azote dans le milieu de culture est compris de 1 à 300,
- le rapport carbone sur phosphore dans le milieu de culture est compris de 80 à 800,
- le milieu de culture de ladite souche comprend :

  ◦ de l'huile de colza, de l'acide oléique ou du glycérol, à une concentration comprise de 1 à 100 g/l,
  o éventuellement du glucose, à une concentration comprise de 1g à 100g/L, notamment de 1g à 20g/L, en particulier de 1g à 10g/L, plus particulièrement de 1g à 5g/L,
  o NaCl, à une concentration comprise d'environ 100 g/L à environ 300 g/L, notamment d'environ 100 g/L à environ 250 g/L,

o KCl, à une concentration comprise de 0 à 20 g/L, en particulier de 2 g/L à 20 g/L,
◦ $CaCl_2.2H_2O$, à une concentration comprise de 0 à 20 g/L, en particulier de 2 g/L à 20 g/L,
◦ $MgCl_2.6H_2O$, à une concentration comprise de 0 à 20 g/L, en particulier de 2 g/L à 20 g/L,

le rapport massique NaCl/$MgCl_2$ étant compris de 15 à 100, et/ou le rapport massique NaCl/$CaCl_2$ étant compris de 20 à 100.

Selon un mode de réalisation avantageux, l'invention concerne un procédé tel que défini précédemment dans lequel l'étape de croissance de la dite souche est mise en œuvre à une température comprise de 20 à 50°C, notamment environ 37°C.
Selon un mode de réalisation avantageux, l'invention concerne un procédé tel que défini précédemment dans lequel le pH du milieu de culture est compris d'environ pH 5 à environ pH 9, plus particulièrement d'environ pH 6 à environ pH 8, le pH du milieu de culture étant de façon encore plus avantageuse égal à environ pH 7.
Selon un mode de réalisation avantageux, l'invention concerne un procédé tel que défini précédemment, mis en œuvre dans des conditions non stériles.
Selon un mode de réalisation avantageux, l'invention concerne un procédé tel que défini précédemment, mis en œuvre dans des conditions non stériles, dans lequel le milieu de culture comprend au moins un sel, en particulier choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant présent dans le milieu de culture à une concentration comprise d'environ 100 g/L à environ 300 g/L, notamment d'environ 100 g/L à environ 250 g/L.
Selon un mode de réalisation avantageux, l'invention concerne une utilisation telle que définie précédemment, dans laquelle le dit procédé de dégradation :

- d'un substrat constitué de ou comprenant un produit ou un co-produit de raffinerie d'oléagineux ou d'hydrocarbures liquides,
- et éventuellement d'un co-substrat carboné,

est mis en œuvre pour la préparation d'un biopolymère de type polyhydroxyalkanoate (PHA).
Selon un mode de réalisation avantageux, l'invention concerne une utilisation telle que définie précédemment, dans laquelle le dit procédé de dégradation :

- d'un substrat constitué de ou comprenant un élément choisi parmi les triglycérides, leurs sous-produits, les alcanes, les alcènes, les polyènes et leurs mélanges ;
- et éventuellement d'un co-substrat carboné,

est mis en œuvre pour la préparation d'un biopolymère de type polyhydroxyalkanoate (PHA).
Par « polyhydroxyalkanoate », on entend notamment un polymère dont le ou les motifs de répétition sont indépendamment les uns des autres de formule (I) suivante :

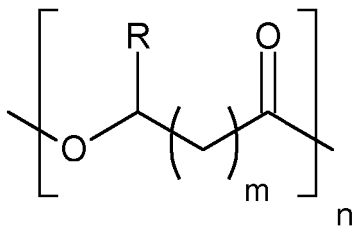
(I)

dans laquelle :

- m est égal à 0, 1, 2, 3 ou 4 ;
- n est notamment compris de 1000 à 20000 ;
- R représente H ou un groupe comprenant de 1 à 20 atomes de carbone, en particulier une chaîne alkyle, plus particulièrement une chaîne alkyle linéaire.

Selon un mode de réalisation avantageux, la présente invention concerne une utilisation telle que définie précédemment, dans laquelle le dit biopolymère de type polyhydroxyalkanoate ne comprend pas de liaison C≡C.
Selon un mode de réalisation avantageux, la présente invention concerne une utilisation telle que définie

précédemment, dans laquelle le dit biopolymère de type polyhydroxyalkanoate est à chaînes latérales courtes.

Par « chaînes latérales courtes », on entend les chaînes méthyle et éthyle.

Par « polyhydroxyalkanoate est à chaînes latérales courtes », on entend notamment les polymères dont le ou les motifs de répétition sont indépendamment les uns des autres de formule (I) telle que définie précédemment, dans laquelle R est un méthyle ou un éthyle.

Selon un mode de réalisation avantageux, la présente invention concerne une utilisation telle que définie précédemment, dans laquelle le dit biopolymère de type poly(3-hydroxybutyrate), poly(3-hydroxyvalérate), ou poly(3-hydroxybutyrate-co-3-hydroxyvalérate).

Le poly(3-hydroxybutyrate) correspond à un polymère dont le motif de répétition est de formule (I) telle que décrite précédemment, dans laquelle m est égal à 1 et R représente un méthyle.

Le poly(3-hydroxyvalérate) correspond à un polymère dont le motif de répétition est de formule (I) telle que décrite précédemment, dans laquelle m est égal à 1 et R représente un éthyle.

Le poly(3-hydroxybutyrate-co-3-hydroxyvalérate) correspond à un copolymère comprenant deux motifs de répétition de formule (I) telle que décrite précédemment, pour lesquels m est égal à 1 et R représente respectivement un méthyle et un éthyle.

Selon un mode de réalisation avantageux, la présente invention concerne une utilisation telle que définie précédemment, dans laquelle le dit biopolymère de type poly(3-hydroxybutyrate-co-3-hydroxyvalérate) de composition molaire hydroxybutyrate/ hydroxyvalérate égale à environ 96/4.

Selon un autre aspect, la présente invention concerne un procédé de préparation d'un biopolymère de type PHA comprenant :

(a) une étape de lyse d'une culture de la souche hyperhalophile aérobie S3S1 déposée le 21 février 2014 à la CNCM sous le numéro CNCM I-4838,
la dite culture étant obtenue après croissance dans un milieu comprenant :

- un substrat constitué de ou comprenant un produit ou un co-produit de raffinerie d'oléagineux ou d'hydrocarbures liquides,
- et éventuellement un co-substrat carboné.

Selon un autre aspect, la présente invention concerne un procédé de préparation d'un biopolymère de type PHA comprenant :

(a) une étape de lyse d'une culture de la souche hyperhalophile aérobie S3S1 déposée le 21 février 2014 à la CNCM sous le numéro CNCM I-4838,
la dite culture étant obtenue après croissance dans un milieu comprenant :

- un substrat constitué de ou comprenant un produit ou un co-produit de raffinerie d'oléagineux ou d'hydrocarbures liquides, en l'absence de co-substrat carboné.

Selon un autre aspect, la présente invention concerne un procédé de préparation d'un biopolymère de type PHA comprenant :

(a) une étape de lyse d'une culture de la souche hyperhalophile aérobie S3S1 déposée le 21 février 2014 à la CNCM sous le numéro CNCM I-4838,
la dite culture étant obtenue après croissance dans un milieu comprenant :

- un substrat constitué de ou comprenant un élément choisi parmi les triglycérides, leurs sous-produits, les alcanes, les alcènes, les polyènes et leurs mélanges ;
- et éventuellement un co-substrat carboné.

Selon un autre aspect, la présente invention concerne un procédé de préparation d'un biopolymère de type PHA comprenant :

(a) une étape de lyse d'une culture de la souche hyperhalophile aérobie S3S1 déposée le 21 février 2014 à la CNCM sous le numéro CNCM I-4838,
la dite culture étant obtenue après croissance dans un milieu comprenant :

- un substrat constitué de ou comprenant un élément choisi parmi les triglycérides, leurs sous-produits, les

alcanes, les alcènes, les polyènes et leurs mélanges, en l'absence de co-substrat carboné.

Par « lyse », on entend la désintégration de la membrane des cellules de la dite souche par un agent physique, chimique ou biologique.

Selon un mode de réalisation avantageux, la présente invention concerne un procédé tel que défini précédemment, dans lequel le dit biopolymère de type polyhydroxyalkanoate ne comprend pas de liaison C≡C.

Selon un mode de réalisation avantageux, la présente invention concerne un procédé tel que défini précédemment, dans lequel le dit biopolymère de type polyhydroxyalkanoate est à chaînes latérales courtes.

Selon un mode de réalisation avantageux, la présente invention concerne un procédé tel que défini précédemment, dans lequel le dit biopolymère de type poly(3-hydroxybutyrate), poly(3-hydroxyvalérate), ou poly(3-hydroxybutyrate-co-3-hydroxyvalérate).

Selon un mode de réalisation avantageux, la présente invention concerne un procédé tel que défini précédemment, dans lequel le dit biopolymère de type poly(3-hydroxybutyrate-co-3-hydroxyvalérate) de composition molaire hydroxybutyrate/ hydroxyvalérate égale à environ 96/4.

Selon un mode de réalisation avantageux, la description concerne un procédé tel que défini précédemment, comprenant une étape de lyse d'une culture d'une souche hyperhalophile aérobie dont le génome total présente un pourcentage d'identité d'au moins 90%, 91%, 92%, 93%, 94%, 95%, 96%, 97%, 98% ou 99% avec le génome total de la dite souche S3S1.

Selon un mode de réalisation avantageux, la description concerne un procédé tel que défini précédemment, comprenant une étape de lyse d'une culture d'une souche hyperhalophile aérobie étant telle que le pourcentage d'hybridation ADN-ADN entre cette souche hyperhalophile aérobie et la souche S3S1 est d'au moins 90%, 91%, 92%, 93%, 94%, 95%, 96%, 97%, 98% ou 99%.

Selon un mode de réalisation avantageux, l'invention concerne un procédé tel que défini précédemment dans lequel l'étape de lyse (a) comprend la mise en contact de la dite culture de la dite souche, optionnellement sous forme lyophilisée, avec un détergent pour obtenir un lysat cellulaire.

Selon un autre mode de réalisation avantageux, l'invention concerne un procédé tel que défini précédemment dans lequel l'étape de lyse (a) comprend la sonication de la dite culture de la dite souche pour obtenir un lysat cellulaire.

Selon un autre mode de réalisation avantageux, l'invention concerne un procédé tel que défini précédemment dans lequel l'étape de lyse (a) comprend la mise en contact de la dite culture de la dite souche, optionnellement sous forme lyophilisée, avec un détergent, puis la sonication du mélange ainsi obtenu pour obtenir un lysat cellulaire.

En particulier, le dit PHA se trouve dans le cytoplasme des cellules de la dite souche.

Le dit détergent est par exemple un tensioactif anionique, en particulier un sel de sodium, de potassium, d'ammonium ou d'alkyl ammonium de sulfates, plus particulièrement ceux obtenus par sulfatation d'alcools comprenant une chaîne alkyle de 8 à 18 atomes de carbone.

Des exemples spécifiques de sulfates d'alkyle utiles dans la présente invention comprennent le dodécylsulfate de sodium, le dodécyl sulfate de potassium, le dodécyl sulfate d'ammonium, le dodécyle sulfate de monoéthanolammonium, le dodécyle sulfate de diéthanolammonium, le dodécyle sulfate de triéthanolammonium, le dodécyle sulfate de tétraéthanolammonium, et le lauryl éther sulfate de sodium.

La sonication est par exemple effectuée à une puissance de 8,1 watts, pendant 3 fois 2 minutes, en particulier à l'aide d'un appareil Vibra-cell Bioblock Scientific 72442.

Selon un mode de réalisation avantageux, l'invention concerne un procédé tel que défini précédemment dans lequel l'étape de lyse (a) est mise en œuvre à une température comprise d'environ 0°C à environ 12°C, en particulier à une température d'environ 4°C.

Selon un mode de réalisation avantageux, l'invention concerne un procédé tel que défini précédemment comprenant une étape (b) d'extraction du PHA à partir du lysat cellulaire.

Selon un mode de réalisation avantageux, l'invention concerne un procédé tel que défini précédemment dans lequel l'étape (b) d'extraction du PHA est mise en œuvre par précipitation en ajoutant de l'hypochlorite de sodium, notamment de l'hypochlorite de sodium à au moins 25 %, en particulier 30%, de chlore actif, dans le culot cellulaire obtenu par centrifugation du lysat cellulaire, en particulier dans un rapport 1 :1 volume à volume, pour obtenir le dit biopolymère de type PHA et une fraction cellulaire.

Selon un mode de réalisation avantageux, l'invention concerne un procédé tel que défini précédemment comprenant (c) une étape de purification du PHA.

Selon un mode de réalisation particulièrement avantageux, l'invention concerne un procédé tel que défini précédemment dans lequel ladite étape de purification du PHA est mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire.

Selon un mode de réalisation particulièrement avantageux, l'invention concerne un procédé tel que défini

précédemment dans lequel ladite étape de purification du PHA est mise en œuvre par lavage dudit biopolymère de type PHA et de ladite fraction cellulaire avec un solvant organique ou un mélange de solvants organiques, par exemple un mélange d'acétone et d'éthanol, puis ajout d'un solvant organique, notamment le chloroforme, en particulier à une température comprise de 20°C à 80°C, plus particulièrement à une température de 45°C à 65°C, pour obtenir une solution de PHA dans ledit solvant organique, ladite solution étant dépourvue de la fraction cellulaire.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM sous le numéro CNCM I-4838, pour obtenir un lysat cellulaire,
une étape (b) d'extraction du PHA à partir du lysat cellulaire,
(c) une étape de purification du PHA.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM sous le numéro CNCM I-4838, pour obtenir un lysat cellulaire,
la dite étape de lyse (a) comprenant :

- la mise en contact de la dite culture de la dite souche, optionnellement sous forme lyophilisée, avec un détergent pour obtenir un lysat cellulaire,
- la sonication de la dite culture de la dite souche pour obtenir un lysat cellulaire, ou
- la mise en contact de la dite culture de la dite souche, optionnellement sous forme lyophilisée, avec un détergent, puis la sonication du mélange ainsi obtenu pour obtenir un lysat cellulaire,

une étape (b) d'extraction du PHA à partir du lysat cellulaire,
la dite étape (b) d'extraction du PHA étant mise en œuvre par précipitation en ajoutant de l'hypochlorite de sodium, notamment de l'hypochlorite de sodium à au moins 25 %, en particulier 30%, de chlore actif, dans le culot cellulaire obtenu par centrifugation du lysat cellulaire, en particulier dans un rapport 1 :1 volume à volume,
pour obtenir le dit biopolymère de type PHA et une fraction cellulaire,
(c) une étape de purification du PHA,

ladite étape de purification du PHA étant en particulier mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire.

Selon un mode de réalisation avantageux, l'invention concerne un procédé tel que défini précédemment dans lequel la dite souche est cultivée sur un milieu comprenant un substrat choisi parmi le groupe constitué :

- des huiles végétales et animales, en particulier les huiles végétales, plus particulièrement l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, et leurs dérivés, en particulier les huiles de friture ;
- des alcanes linéaires, ramifiés ou cycliques, comprenant de 6 à 24 atomes de carbone, en particulier de 10 à 22 atomes de carbone ;
- des alcènes linéaires, ramifiés ou cycliques, comprenant de 6 à 24 atomes de carbone, en particulier de 10 à 22 atomes de carbone ;
- des polyènes linéaires, ramifiés ou cycliques, comprenant de 6 à 24 atomes de carbone, en particulier de 10 à 22 atomes de carbone ;
- des acides gras, en particulier les acides gras saturés et insaturés, plus particulièrement les acides gras mono insaturés, di insaturés et poly insaturés, lesdits acides gras comprenant un acide carboxylique et une chaîne comprenant de 6 à 24 atomes de carbone, en particulier de 10 à 22 atomes de carbone, ainsi que leurs dérivés, en particulier les esters des dits acides gras, plus particulièrement les esters méthyliques et éthyliques des dits acides gras;
- des trigycérides ;
- du glycérol ;
- des co-produits des raffineries d'huile, en particulier d'huile végétale, notamment les pâtes de neutralisation, les

condensats de désodorisation, les gommes, les distillats d'acides gras, les graisses d'aéroflottateurs et les boues de station d'épuration,.

Selon un mode de réalisation avantageux, l'invention concerne un procédé tel que défini précédemment, dans lequel ledit substrat est :

- une huile végétale, en particulier l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, plus particulièrement l'huile de colza ou l'huile de tournesol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, α-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, α-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- le glycérol,

ou l'un de leurs mélanges.

Selon un mode de réalisation avantageux, l'invention concerne un procédé tel que défini précédemment dans lequel la dite souche est cultivée sur un milieu comprenant un substrat et un co-substrat carboné.

Selon un mode de réalisation avantageux, l'invention concerne un procédé tel que défini précédemment dans lequel la dite souche est cultivée sur un milieu comprenant un substrat et un co-substrat carboné, ledit co-substrat étant un sucre, en particulier le glucose.

Selon un mode de réalisation avantageux, l'invention concerne un procédé tel que défini précédemment dans lequel la dite souche est cultivée sur un milieu comprenant un substrat et un co-substrat carboné, ledit co-substrat étant en particulier un sucre, notamment le glucose, la concentration en co-substrat étant comprise de 1g à 100g/L, notamment de 1g à 20g/L, en particulier de 1g à 10g/L, plus particulièrement de 1g à 5g/L.

Selon un mode de réalisation avantageux, l'invention concerne un procédé tel que défini précédemment dans lequel la dite souche est cultivée sur un milieu comprenant :

❖ un substrat choisi parmi le groupe constitué :

- des huiles végétales ou animales, en particulier les huiles végétales, plus particulièrement l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, et leurs dérivés, en particulier les huiles de friture ;
- des alcanes linéaires, ramifiés ou cycliques, comprenant de 6 à 24 atomes de carbone, en particulier de 10 à 22 atomes de carbone ;
- des alcènes linéaires, ramifiés ou cycliques, comprenant de 6 à 24 atomes de carbone, en particulier de 10 à 22 atomes de carbone ;
- des polyènes linéaires, ramifiés ou cycliques, comprenant de 6 à 24 atomes de carbone, en particulier de 10 à 22 atomes de carbone ;
- des acides gras, en particulier les acides gras saturés et insaturés, plus particulièrement les acides gras mono insaturés, di insaturés et poly insaturés, lesdits acides gras comprenant un acide carboxylique et une chaîne comprenant de 6 à 24 atomes de carbone, en particulier de 10 à 22 atomes de carbone, ainsi que leurs dérivés, en particulier les esters des dits acides gras, plus particulièrement les esters méthyliques et éthyliques des dits acides gras;
- des trigycérides ;
- du glycérol ;
- des co-produits des raffineries d'huile, en particulier d'huile végétale, notamment les pâtes de neutralisation, les condensats de désodorisation, les gommes, les distillats d'acides gras, les graisses d'aéroflottateurs et les

boues de station d'épuration,

et leurs mélanges,

❖ un co-substrat carboné, ledit co-substrat étant un sucre, en particulier le glucose,

contenus dans le milieu de culture de la dite souche hyperhalophile aérobie.

Selon un mode de réalisation avantageux, l'invention concerne un procédé tel que défini précédemment dans lequel la dite souche est cultivée sur un milieu comprenant :

❖ un substrat, ledit substrat étant:

- une huile végétale, en particulier l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, plus particulièrement l'huile de colza ou l'huile de tournesol,
- un acide gras saturé ou insaturé choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- un triglycéride constitué de glycérol dont les trois groupements hydroxyle sont estérifiés par des acides gras choisi parmi l'acide caprylique, caprique, laurique, myristique, palmitique, stéarique, arachidique, béhénique, lignocérique, cérotique sapiénique, élaïdique, vaccénique, linoélaïdique, $\alpha$-linolénique, érucique, docosahexaénoïque, oléique, linoléique, arachidonique, eicosapentaénoïque, hexadecatriénoïque, stéaridonique, eicosatriénoïque, heneicosapentaénoïque, docosapentaénoïque, tetracosapentaénoïque, tetracosahexaénoïque, gamma-linolénique, eicosadiénoïque, docosadiénoïque, adrénique, tetracosatetraénoïque, eicosenoïque, pinolénique, podocarpique et myristoléique,
- le glycérol,

ou l'un de leurs mélanges,

❖ un co-substrat carboné, ledit co-substrat étant un sucre, en particulier le glucose,

contenus dans le milieu de culture de la dite souche hyperhalophile aérobie.

Selon un mode de réalisation avantageux, l'invention concerne un procédé tel que défini précédemment dans lequel la dite souche est cultivée dans un milieu comprenant un substrat et éventuellement un co-substrat carboné choisis parmi du glycérol et du stéarate, du glycérol et du stéarate de méthyle, du glycérol et de l'acide oléique, du glycérol et de l'oléate de méthyle, du glycérol et de l'acide érucique, du glycérol et de l'érucate de méthyle, du glycérol et de l'acide laurique, du glycérol et du laurate de méthyle, du glycérol et de l'huile, du glycérol et de l'huile de colza, du glycérol et de l'huile de tournesol, du glucose et du stéarate, du glucose et du stéarate de méthyle, du glucose et de l'acide oléique, du glucose et de l'oléate de méthyle, du glucose et de l'acide érucique, du glucose et de l'érucate de méthyle, du glucose et de l'acide laurique, du glucose et du laurate de méthyle, du glucose et de l'huile, du glucose et de l'huile de colza, ou du glucose et de l'huile de tournesol.

Selon un mode de réalisation avantageux, l'invention concerne un procédé tel que défini précédemment dans lequel la dite souche est cultivée dans un milieu comprenant un moins un sel.

Selon un mode de réalisation avantageux, l'invention concerne un procédé tel que défini précédemment dans lequel le sel est choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment dans lequel la dite souche est cultivée dans un milieu dans lequel le rapport massique $NaCl/MgCl_2$ est compris de 15 à 100.

Selon un mode de réalisation avantageux, l'invention concerne un procédé tel que défini précédemment dans lequel la dite souche est cultivée dans un milieu dans lequel le rapport massique $NaCl/CaCl_2$ est compris de 20 à 100.

Lorsque, dans le milieu de culture, le rapport massique $NaCl/MgCl_2$ est compris de 15 à 100, et/ou le rapport massique $NaCl/CaCl_2$ est compris de 20 à 100, la croissance de la souche est améliorée par rapport à celle de la même souche dans un milieu dans lequel le rapport massique $NaCl/MgCl_2$ n'est pas compris de 15 à 100, et/ou dans lequel le rapport massique $NaCl/CaCl_2$ n'est pas compris de 20 à 100, en particulier un milieu ne comprenant pas de $MgCl_2$ et/ou de $CaCl_2$.

Selon un mode de réalisation avantageux, l'invention concerne un procédé tel que défini précédemment dans lequel la dite souche est cultivée sous une atmosphère comprenant moins de 21% en volume de dioxygène, en particulier

moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène.

Selon un mode de réalisation avantageux, l'invention concerne un procédé tel que défini précédemment dans lequel le rapport carbone sur azote dans le milieu de culture est compris de 1 à 300.

Lorsque le rapport carbone sur azote dans le milieu de culture est compris de 1 à 300, la dite souche peut être en conditions de stress, et la quantité de PHA produite peut alors être augmentée.

Selon un mode de réalisation avantageux, l'invention concerne un procédé tel que défini précédemment dans lequel le rapport carbone sur phosphore dans le milieu de culture est compris de 80 à 800.

Lorsque le rapport carbone sur phosphore dans le milieu de culture est compris de 80 à 800, la dite souche peut être en conditions de stress, et la quantité de PHA produite peut alors être augmentée.

Selon un mode de réalisation avantageux, l'invention concerne un procédé tel que défini précédemment, mis en œuvre dans des conditions non stériles.

Selon un mode de réalisation avantageux, l'invention concerne un procédé tel que défini précédemment, mis en œuvre dans des conditions non stériles, dans lequel le milieu de culture comprend au moins un sel, en particulier choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant présent dans le milieu de culture à une concentration comprise d'environ 100 g/L à environ 300 g/L, notamment d'environ 100 g/L à environ 250 g/L.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM sous le numéro CNCM I-4838, dans un milieu de culture, pour obtenir un lysat cellulaire,
la dite étape de lyse (a) comprenant :

- la mise en contact de la dite culture de la dite souche, optionnellement sous forme lyophilisée, avec un détergent pour obtenir un lysat cellulaire,
- la sonication de la dite culture de la dite souche pour obtenir un lysat cellulaire, ou
- la mise en contact de la dite culture de la dite souche, optionnellement sous forme lyophilisée, avec un détergent, puis la sonication du mélange ainsi obtenu pour obtenir un lysat cellulaire,

la dite étape (b) d'extraction du PHA étant mise en œuvre par précipitation en ajoutant de l'hypochlorite de sodium, notamment de l'hypochlorite de sodium à au moins 25 %, en particulier 30%, de chlore actif, dans le culot cellulaire obtenu par centrifugation du lysat cellulaire, en particulier dans un rapport 1 :1 volume à volume,
pour obtenir le dit biopolymère de type PHA et une fraction cellulaire,
(c) une étape de purification du PHA,
ladite étape de purification du PHA étant en particulier mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire,
et dans lequel le milieu de culture de ladite souche comprend :

o de l'huile de colza, de l'acide oléique ou du glycérol, à une concentration comprise de 1 à 100 g/l,
o éventuellement du glucose, à une concentration comprise de 1g à 100g/L, notamment de 1g à 20g/L, en particulier de 1g à 10g/L, plus particulièrement de 1g à 5g/L,
◦ NaCl, à une concentration comprise d'environ 100 g/L à environ 300 g/L, notamment d'environ 100 g/L à environ 250 g/L,
◦ KCl, à une concentration comprise de 0 à 20 g/L, en particulier de 2 g/L à 20 g/L,
◦ $CaCl_2.2H_2O$, à une concentration comprise de 0 à 20 g/L, en particulier de 2 g/L à 20 g/L,
◦ $MgCl_2.6H_2O$, à une concentration comprise de 0 à 20 g/L, en particulier de 2 g/L à 20 g/L,

le rapport massique $NaCl/MgCl_2$ étant compris de 15 à 100, et/ou le rapport massique $NaCl/CaCl_2$ étant compris de 20 à 100.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM sous le numéro CNCM I-4838, dans un milieu de culture, pour obtenir un lysat cellulaire,

la dite étape de lyse (a) comprenant :

- la mise en contact de la dite culture de la dite souche, optionnellement sous forme lyophilisée, avec un détergent pour obtenir un lysat cellulaire,
- la sonication de la dite culture de la dite souche pour obtenir un lysat cellulaire, ou
- la mise en contact de la dite culture de la dite souche, optionnellement sous forme lyophilisée, avec un détergent, puis la sonication du mélange ainsi obtenu pour obtenir un lysat cellulaire,

la dite étape (b) d'extraction du PHA étant mise en œuvre par précipitation en ajoutant de l'hypochlorite de sodium, notamment de l'hypochlorite de sodium à au moins 25 %, en particulier 30%, de chlore actif, dans le culot cellulaire obtenu par centrifugation du lysat cellulaire, en particulier dans un rapport 1 :1 volume à volume,
pour obtenir le dit biopolymère de type PHA et une fraction cellulaire,
(c) une étape de purification du PHA,
ladite étape de purification du PHA étant en particulier mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire,
et dans lequel :

- la dite souche est cultivée sous une atmosphère comprenant moins de 21% en volume de dioxygène, en particulier moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène,
- le rapport carbone sur azote dans le milieu de culture est compris de 1 à 300,
- le rapport carbone sur phosphore dans le milieu de culture est compris de 80 à 800,
- le milieu de culture de ladite souche comprend :

    - de l'huile de colza de l'acide oléique ou du glycérol, à une concentration comprise de 1 à 100 g/l,
    - éventuellement du glucose, à une concentration comprise de 1g à 100g/L, notamment de 1g à 20g/L, en particulier de 1g à 10g/L, plus particulièrement de 1g à 5g/L,
    - NaCl, à une concentration comprise d'environ 100 g/L à environ 300 g/L, notamment d'environ 100 g/L à environ 250 g/L,
    - KCl, à une concentration comprise de 0 à 20 g/L, en particulier de 2 g/L à 20 g/L,
    - $CaCl_2.2H_2O$, à une concentration comprise de 0 à 20 g/L, en particulier de 2 g/L à 20 g/L,
    - $MgCl_2.6H_2O$, à une concentration comprise de 0 à 20 g/L, en particulier de 2 g/L à 20 g/L,

le rapport massique $NaCl/MgCl_2$ étant compris de 15 à 100, et/ou le rapport massique $NaCl/CaCl_2$ étant compris de 20 à 100.

Selon un autre aspect, un PHA susceptible d'être obtenu à partir d'un procédé tel que défini précédemment est décrit.
Egalement décrit est un PHA ayant un indice de polymolécularité compris de 1,0 à 2,0, en particulier de 1,5 à 1,9, plus particulièrement de 1,6 à 1,8, notamment d'environ 1,7.
Selon un autre aspect, la description concerne un PHA de masse moléculaire en poids supérieure à 500 000 g/mol, en particulier supérieure à 500 000 g/mol, 600 000 g/mol, 700 000 g/mol, 800 000 g/mol, 900 000 g/mol, ou 1 000 000 g/mol.
Selon un autre aspect, la description concerne un PHA de masse moléculaire en poids supérieure à 500 000 g/mol, en particulier supérieure à 500 000 g/mol, 600 000 g/mol, 700 000 g/mol, 800 000 g/mol, 900 000 g/mol, ou 1 000 000 g/mol, caractérisé par de courtes chaines latérales.
Selon un autre aspect, la description concerne un PHA de masse moléculaire en poids supérieure à 500 000 g/mol, en particulier supérieure à 500 000 g/mol, 600 000 g/mol, 700 000 g/mol, 800 000 g/mol, 900 000 g/mol, ou 1 000 000 g/mol, ledit PHA ayant un indice de polymolécularité compris de 1,0 à 2,0, en particulier de 1,5 à 1,9, plus particulièrement de 1,6 à 1,8, notamment d'environ 1,7.
Selon un autre aspect, la description concerne un PHA de masse moléculaire en poids supérieure à 500 000 g/mol, en particulier supérieure à 500 000 g/mol, 600 000 g/mol, 700 000 g/mol, 800 000 g/mol, 900 000 g/mol, ou 1 000 000 g/mol, caractérisé par de courtes chaines latérales, ledit PHA ayant un indice de polymolécularité compris de 1,0 à 2,0, en particulier de 1,5 à 1,9, plus particulièrement de 1,6 à 1,8, notamment d'environ 1,7.
Selon un autre aspect, la description concerne un PHA de masse moléculaire en poids supérieure à 500 000 g/mol, en particulier supérieure à 500 000 g/mol, 600 000 g/mol, 700 000 g/mol, 800 000 g/mol, 900 000 g/mol, ou 1

000 000 g/mol, caractérisé par de courtes chaines latérales et un indice de transparence donné.

Selon un autre aspect, la description concerne un PHA de masse moléculaire en nombre supérieure à 400 000 g/mol, en particulier supérieure à 450 000 g/mol, 500 000 g/mol, 550 000 g/mol, ou 600 000 g/mol.

Selon un autre aspect, la description concerne un PHA de masse moléculaire en nombre supérieure à 400 000 g/mol, en particulier supérieure à 450 000 g/mol, 500 000 g/mol, 550 000 g/mol, ou 600 000 g/mol, caractérisé par de courtes chaines latérales.

Selon un autre aspect, la description concerne un PHA de masse moléculaire en nombre supérieure à 400 000 g/mol, en particulier supérieure à 450 000 g/mol, 500 000 g/mol, 550 000 g/mol, ou 600 000 g/mol, ledit PHA ayant un indice de polymolécularité compris de 1,0 à 2,0, en particulier de 1,5 à 1,9, plus particulièrement de 1,6 à 1,8, notamment d'environ 1,7.

Selon un autre aspect, la description concerne un PHA de masse moléculaire en nombre supérieure à 400 000 g/mol, en particulier supérieure à 450 000 g/mol, 500 000 g/mol, 550 000 g/mol, ou 600 000 g/mol, caractérisé par de courtes chaines latérales, ledit PHA ayant un indice de polymolécularité compris de 1,0 à 2,0, en particulier de 1,5 à 1,9, plus particulièrement de 1,6 à 1,8, notamment d'environ 1,7.

Selon un autre aspect, la description concerne un PHA de masse moléculaire en nombre supérieure à 400 000 g/mol, en particulier supérieure à 450 000 g/mol, 500 000 g/mol, 550 000 g/mol, ou 600 000 g/mol, caractérisé par de courtes chaines latérales et un indice de transparence donné.

Par « chaînes latérales courtes », on entend les chaînes méthyle et éthyle.

La masse molaire moyenne en poids, la masse molaire moyenne en nombre et l'indice de polymolécularité des PHA de l'invention peuvent être déterminées par chromatographie sur gel perméable (GPC), avec notamment un réfractomètre différentiel (RI) ou la diffusion de lumière (ddl) comme mode de détection.

La transparence d'une solution de PHA peut être déterminée par turbidité. La turbidité est une propriété intrinsèque des matériaux qui est liée à l'atténuation de l'intensité de la lumière lorsqu'elle traverse des matériaux.

Les valeurs de l'absorbance (Abs) peuvent être déterminées à l'aide d'un spectrophotomètre Varian, Cary 60 et la turbidité est alors calculée de la façon suivante :

$$\text{Turbidité T} = 1 - 10^{(\text{-Abs})}.$$

Selon un mode de réalisation avantageux, la description concerne un PHA de type poly(3-hydroxybutyrate), poly(3-hydroxyvalérate), ou poly(3-hydroxybutyrate-co-3-hydroxyvalérate).

Selon un mode de réalisation avantageux, la description concerne un PHA de type poly(3-hydroxybutyrate-co-3-hydroxyvalérate) de composition molaire hydroxybutyrate/ hydroxyvalérate égale à environ 96/4.

Selon un mode de réalisation avantageux, la description concerne un PHA tel que défini précédemment comprenant ou consistant en un biopolymère de motif P(HB-co-HV) en proportion 96/4,
le dit polymère ayant un indice de polydispersité compris de 1,0 à 2,0, en particulier de 1,5 à 1,9, plus particulièrement de 1,6 à 1,8, notamment d'environ 1,7,
une Tg comprise de -10°C à +10°C, en particulier de -5°C à +10°C, notamment d'environ +5°C,
une Tf supérieure ou égale à 140°, en particulier comprise de 140°C à 170°C, plus particulièrement de 145°C à 165°C, notamment d'environ 157°C.

La température de transition vitreuse (Tg) et la température de fusion (Tf) peuvent être déterminées par calorimétrie différentielle à balayage (DSC). La température de fusion peut être déterminée au 1$^{er}$ passage et la température de transition vitreuse au 2$^{ème}$ passage.

En particulier, la température de transition vitreuse (Tg) et la température de fusion (Tf) peuvent être déterminées par DSC, sur un appareil Diamon DSC (Perkin Elmer) : la température de fusion est par exemple déterminée au premier passage et la température de transition vitreuse au deuxième passage selon le programme de température suivant : 1) de 20°C à -50°C à 40°C/min ; 2) 3 min à -50°C ; 3) premier passage : de -50°C à 200°C à 20°C/min ; 4) de 180°C à -50°C à 200°C/min ; 5) 3 min à -50°C ; 6) deuxième passage : - 50°C à 200°C à 20°C/min.

Les solutions de PHA dans un solvant, en particulier organique, sont parfaitement limpides, et très visqueuses.

Les mesures de viscosité peuvent être faites soit par un rhéomètre (TA Instrument, HR2) soit par un viscosimètre à chute de bille, si la solution est plus fluide (Lovis, Anton Paar). Les mesures rhéologiques peuvent être réalisées soit par écoulement soit par cisaillement et permettent d'accéder aux modules de perte et de conservation ainsi qu'aux valeurs de la viscosité dynamique.

Selon un autre aspect, l'invention concerne une souche hyperhalophile aérobie S3S1 déposée le 21 février 2014 à la CNCM sous le numéro CNCM I-4838.

Selon un autre aspect, l'invention concerne une utilisation de la souche hyperhalophile aérobie S3S1 déposée le 21 février 2014 à la CNCM sous le numéro CNCM I-4838 pour la mise en œuvre d'un procédé de dégradation d'un

substrat carboné comprenant du glucose.

Selon un autre aspect, la description concerne une utilisation de la souche hyperhalophile aérobie S3S1 déposée le 21 février 2014 à la CNCM sous le numéro CNCM I-4838, ou d'une souche hyperhalophile aérobie étant telle que le pourcentage d'hybridation ADN-ADN entre cette souche hyperhalophile aérobie et la souche S3S1 est d'au moins 90%, en particulier d'au moins 92%,

pour la mise en œuvre d'un procédé de dégradation d'un substrat carboné comprenant du glucose.

Selon un autre aspect, l'invention concerne une utilisation de la souche hyperhalophile aérobie S3S1 déposée le 21 février 2014 à la CNCM sous le numéro CNCM I-4838,

pour la mise en œuvre d'un procédé de dégradation d'un substrat carboné en l'absence du glucose.

Selon un mode de réalisation avantageux, l'invention concerne une utilisation telle que définie précédemment, dans laquelle le dit procédé de dégradation d'un substrat carboné comprenant du glucose est mis en œuvre pour la préparation d'un biopolymère de type polyhydroxyalkanoate (PHA).

Selon un autre aspect, l'invention concerne un procédé de dégradation d'un substrat carboné de type huile, triglycérides, glycérol, acides gras ou leurs dérivés, comprenant :

une étape de croissance d'une souche hyperhalophile aérobie S3S1 déposée le 21 février 2014 à la CNCM sous le numéro CNCM I-4838, dans un milieu de culture comprenant du glucose.

Selon un autre aspect, l'invention concerne un procédé de dégradation d'un substrat carboné de type huile, triglycérides, glycérol, acides gras ou leurs dérivés, comprenant :

une étape de croissance d'une souche hyperhalophile aérobie S3S1 déposée le 21 février 2014 à la CNCM sous le numéro CNCM I-4838, dans un milieu de culture en l'absence du glucose.

Selon un autre aspect, l'invention concerne un procédé de préparation d'un biopolymère de type PHA comprenant :

(a) une étape de lyse d'une culture de la souche hyperhalophile aérobie S3S1 déposée le 21 février 2014 à la CNCM sous le numéro CNCM I-4838,

la dite culture étant obtenue après croissance dans un milieu comprenant du glucose.

Selon un autre aspect, l'invention concerne un procédé de préparation d'un biopolymère de type PHA comprenant :

(a) une étape de lyse d'une culture de la souche hyperhalophile aérobie S3S1 déposée le 21 février 2014 à la CNCM sous le numéro CNCM I-4838,

la dite culture étant obtenue après croissance dans un milieu en l'absence du glucose.

Selon un mode de réalisation avantageux, la description concerne un procédé tel que défini précédemment, comprenant une étape de croissance d'une souche hyperhalophile aérobie dont le génome total présente un pourcentage d'identité d'au moins 90%, 91%, 92%, 93%, 94%, 95%, 96%, 97%, 98% ou 99% avec le génome total de la dite souche S3S1.

Selon un mode de réalisation avantageux, la description concerne un procédé tel que défini précédemment, comprenant une étape de croissance d'une souche hyperhalophile aérobie étant telle que le pourcentage d'hybridation ADN-ADN entre cette souche hyperhalophile aérobie et la souche S3S1 est d'au moins 90%, 91%, 92%, 93%, 94%, 95%, 96%, 97%, 98% ou 99%.

Selon un mode de réalisation avantageux, l'invention concerne un procédé tel que défini précédemment, dans lequel le milieu de culture comprend au moins un sel.

Selon un mode de réalisation avantageux, l'invention concerne un procédé tel que défini précédemment, dans lequel le sel est choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges.

Selon un mode de réalisation avantageux, l'invention concerne un procédé tel que défini précédemment, dans lequel ledit sel est présent dans le milieu de culture à une concentration comprise d'environ 100 g/L à environ 300 g/L, notamment d'environ 100 g/L à environ 250 g/L.

Selon un mode de réalisation avantageux, l'invention concerne un procédé tel que défini précédemment dans lequel la dite souche est cultivée dans un milieu dans lequel le rapport massique $NaCl/MgCl_2$ est compris de 15 à 100.

Selon un mode de réalisation avantageux, l'invention concerne un procédé tel que défini précédemment dans lequel la dite souche est cultivée dans un milieu dans lequel le rapport massique $NaCl/CaCl_2$ est compris de 20 à 100.

Selon un mode de réalisation avantageux, l'invention concerne un procédé tel que défini précédemment dans lequel la dite souche est cultivée sous une atmosphère comprenant moins de 21% en volume de dioxygène, en particulier moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène.

Selon un mode de réalisation avantageux, l'invention concerne un procédé tel que défini précédemment dans lequel le rapport carbone sur azote dans le milieu de culture est compris de 1 à 300.

Selon un mode de réalisation avantageux, l'invention concerne un procédé tel que défini précédemment dans lequel le rapport carbone sur phosphore dans le milieu de culture est compris de 80 à 800.

Selon un mode de réalisation avantageux, l'invention concerne un procédé tel que défini précédemment dans lequel la dite souche est cultivée dans un milieu dans lequel :

- le rapport massique $NaCl/MgCl_2$ est compris de 15 à 100, et/ou
- le rapport massique $NaCl/CaCl_2$ est compris de 20 à 100.

Selon un mode de réalisation avantageux, l'invention concerne un procédé tel que défini précédemment dans lequel :

- la dite souche est cultivée dans un milieu dans lequel le rapport massique $NaCl/MgCl_2$ est compris de 15 à 100, et/ou le rapport massique $NaCl/CaCl_2$ est compris de 20 à 100,
- la dite souche est cultivée sous une atmosphère comprenant moins de 21% en volume de dioxygène, en particulier moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène,
- le rapport carbone sur azote dans le milieu de culture est compris de 1 à 300,

le rapport carbone sur phosphore dans le milieu de culture est compris de 80 à 800.

Selon un mode de réalisation avantageux, l'invention concerne un procédé tel que défini précédemment, mis en œuvre dans des conditions non stériles.

Selon un mode de réalisation avantageux, l'invention concerne un procédé tel que défini précédemment, mis en œuvre dans des conditions non stériles, dans lequel le milieu de culture comprend au moins un sel, en particulier choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges, ledit sel étant présent dans le milieu de culture à une concentration comprise d'environ 100 g/L à environ 300 g/L, notamment d'environ 100 g/L à environ 250 g/L.

Selon un mode de réalisation avantageux, l'invention concerne un procédé tel que défini précédemment dans lequel l'étape de lyse (a) comprend la mise en contact de la dite culture de la dite souche, optionnellement sous forme lyophilisée, avec un détergent pour obtenir un lysat cellulaire.

Selon un autre mode de réalisation avantageux, l'invention concerne un procédé tel que défini précédemment dans lequel l'étape de lyse (a) comprend la sonication de la dite culture de la dite souche pour obtenir un lysat cellulaire.

Selon un autre mode de réalisation avantageux, l'invention concerne un procédé tel que défini précédemment dans lequel l'étape de lyse (a) comprend la mise en contact de la dite culture de la dite souche, optionnellement sous forme lyophilisée, avec un détergent, puis la sonication du mélange ainsi obtenu pour obtenir un lysat cellulaire.

Selon un mode de réalisation avantageux, l'invention concerne un procédé tel que défini précédemment dans lequel l'étape (b) d'extraction du PHA est mise en œuvre

par précipitation en ajoutant de l'hypochlorite de sodium, notamment de l'hypochlorite de sodium à au moins 25 %, en particulier 30%, de chlore actif, dans le culot cellulaire obtenu par centrifugation du lysat cellulaire, en particulier dans un rapport 1 :1 volume à volume, pour obtenir le dit biopolymère de type PHA et une fraction cellulaire.

Selon un mode de réalisation avantageux, l'invention concerne un procédé tel que défini précédemment comprenant (c) une étape de purification du PHA.

Selon un mode de réalisation particulièrement avantageux, l'invention concerne un procédé tel que défini précédemment dans lequel ladite étape de purification du PHA est mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire.

Selon un mode de réalisation particulièrement avantageux, l'invention concerne un procédé tel que défini précédemment dans lequel ladite étape de purification du PHA est mise en œuvre par lavage dudit biopolymère de type PHA et de ladite fraction cellulaire avec un solvant organique ou un mélange de solvants organiques, par exemple un mélange d'acétone et d'éthanol, puis ajout d'un solvant organique, notamment le chloroforme, en particulier à une température comprise de 20°C à 80°C, plus particulièrement à une température de 45°C à 65°C, pour obtenir une solution de PHA dans ledit solvant organique, ladite solution étant dépourvue de la fraction cellulaire.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM

sous le numéro CNCM I-4838, pour obtenir un lysat cellulaire,
une étape (b) d'extraction du PHA à partir du lysat cellulaire,
(c) une étape de purification du PHA.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM sous le numéro CNCM I-4838, pour obtenir un lysat cellulaire,
la dite étape de lyse (a) comprenant :

- la mise en contact de la dite culture de la dite souche, optionnellement sous forme lyophilisée, avec un détergent pour obtenir un lysat cellulaire,
- la sonication de la dite culture de la dite souche pour obtenir un lysat cellulaire, ou
- la mise en contact de la dite culture de la dite souche, optionnellement sous forme lyophilisée, avec un détergent, puis la sonication du mélange ainsi obtenu pour obtenir un lysat cellulaire,

une étape (b) d'extraction du PHA à partir du lysat cellulaire,
la dite étape (b) d'extraction du PHA étant mise en œuvre par précipitation en ajoutant de l'hypochlorite de sodium, notamment de l'hypochlorite de sodium à au moins 25 %, en particulier 30%, de chlore actif, dans le culot cellulaire obtenu par centrifugation du lysat cellulaire, en particulier dans un rapport 1 :1 volume à volume,
pour obtenir le dit biopolymère de type PHA et une fraction cellulaire,
(c) une étape de purification du PHA,

ladite étape de purification du PHA étant en particulier mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM sous le numéro CNCM I-4838, pour obtenir un lysat cellulaire,
une étape (b) d'extraction du PHA à partir du lysat cellulaire,
(c) une étape de purification du PHA.

Selon un mode de réalisation avantageux, l'invention concerne un procédé tel que défini précédemment comprenant :

(a) une étape de lyse d'une culture de la souche hyperhalophile aérobie S3S1 déposée le 21 février 2014 à la CNCM sous le numéro CNCM I-4838, ou d'une souche hyperhalophile aérobie étant telle que le pourcentage d'hybridation ADN-ADN entre cette souche hyperhalophile aérobie et la souche S3S1 est d'au moins 90%, en particulier d'au moins 92%, pour obtenir un lysat cellulaire,
la dite étape de lyse (a) comprenant :

- la mise en contact de la dite culture de la dite souche, optionnellement sous forme lyophilisée, avec un détergent pour obtenir un lysat cellulaire,
- la sonication de la dite culture de la dite souche pour obtenir un lysat cellulaire, ou
- la mise en contact de la dite culture de la dite souche, optionnellement sous forme lyophilisée, avec un détergent, puis la sonication du mélange ainsi obtenu pour obtenir un lysat cellulaire,

une étape (b) d'extraction du PHA à partir du lysat cellulaire,
la dite étape (b) d'extraction du PHA étant mise en œuvre par précipitation en ajoutant de l'hypochlorite de sodium, notamment de l'hypochlorite de sodium à au moins 25 %, en particulier 30%, de chlore actif, dans le culot cellulaire obtenu par centrifugation du lysat cellulaire, en particulier dans un rapport 1 :1 volume à volume,
pour obtenir le dit biopolymère de type PHA et une fraction cellulaire,
(c) une étape de purification du PHA,

ladite étape de purification du PHA étant en particulier mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire.

**FIGURES**

La **figure 1** illustre un exemple de pigmentation au rouge de Nil, montrant la présence de PHA dans la souche S3S1.
La **figure 2** présente le spectre RMN $^{1}H$ du PHA obtenu à partir du substrat 3 (Huile de colza + Glucose, exemple Ibis).
La **figure 3** présente le spectre RMN $^{13}C$ du PHA obtenu à partir du substrat 3 (Huile de colza + Glucose, exemple Ibis).

**EXEMPLES**

**Exemple 1 : Dégradation d'un substrat carboné**

Les milieux de culture utilisés pour la dégradation du substrat défini ci-après sont par exemple de la composition suivante :

- $NH_4Cl$, 1g/L;
- KCl, 2g/L ;
- $CaCl_2.2H_2O$, 2g/L ;
- $MgCl_2.6H_2O$, 3g/L ;
- NaCl, 150g/L ;
- extrait de levure, 1g/L ;
- substrat:

  **(1)** Glucose 2g/L,
  **(2)** Glucose 2g/L + 10g/L glycérol,
  **(3)** Glucose 2g/L + 10g/L huile de colza,
  **(4)** Glucose 2g/l+ 10g/L d'acide oléique,
  **(5)** Glycérol 10g/L,
  **(6)** Huile de colza 10g/L, ou
  **(7)** acide oléique 10g/L.

Les pré-cultures ont notamment été réalisées dans des flacons pénicilline contenant 50 ml de milieu de culture.
De plus grands volumes de milieu de culture (1,5 L, voire même 2 L) one également été utilisés.
La culture a été réalisée en aérobiose, à pH 7 et 37°C.

**Exemple 1bis : Production de PHA**

**• Milieux de culture utilisés pour la production de PHA:**

Les milieux de culture utilisés pour la production de PHA sont par exemple de la composition suivante :

- $NH_4Cl$, 1g/L;
- KCl, 2g/L ;
- $CaCl_2.2H_2O$, 2g/L ;
- $MgCl_2.6H_2O$, 3g/L ;
- NaCl, 150g/L ;
- extrait de levure, 1g/L ;
- substrat:

  (1) Glucose 2g/L,
  (2) Glucose 2g/L + 10g/L glycérol,
  (3) Glucose 2g/L + 10g/L huile de colza,
  (4) Glucose 2g/l+ 10g/L d'acide oléique,

(5) Glycérol 10g/L,
(6) Huile de colza 10g/L, ou
(7) acide oléique 10g/L.

Des concentrations plus élevées en substrat peuvent également être utilisées.
Les pré-cultures ont notamment été réalisées dans des flacons pénicilline contenant 50 ml de milieu de culture.
De plus grands volumes de milieu de culture (1,5 L, voire même 2 L) one également été utilisés.
La culture a été réalisée en aérobiose, à pH 7 et 37°C.

**• Détection des polyhydroxyalcanoates (PHA)**

La détection de PHA dans la souche productrice S3S1 a en particulier été réalisée sur un milieu contenant 15% de NaCl ; $NH_4Cl$, 1g/l; KCl, 2 g/l; $CaCl_2.2H_2O$, 2 g/l; $MgCl_2.6H_2O$, 3 g/l; 10 g/l, glycérol ; 1 g/l, d'extrait de levure, en présence ou en absence de glucose. Le pH du milieu est 7.0 et la température de croissance est de 37 °C.
Une solution de rouge de Nil 0,5 μg/ml, filtrée et mise à l'obscurité, a été pulvérisée sur la souche dans le but de détecter les PHA. L'observation directe des cellules bactériennes contenant des granules de PHA a été réalisée par un microscope à contraste de phase: Nikon optiphot (Nikon, Tokyo, Japon) relié à un appareil photo Nikon DS-FI 1, et mis sous fluorescence à 490 nm **(figure 1).**
De façon analogue, la détection des PHA à partir d'un milieu comprenant en tant que substrat de l'huile de colza ou de l'acide oléique, en présence ou en absence de glucose, a été réalisée.

**Exemple 2 : extraction du PHA**

L'extraction de PHA a été réalisée à partir d'un isolat obtenu à l'exemple Ibis, selon le protocole suivant :

- Lyophiliser la culture bactérienne (biomasse) après récupération par centrifugation à 9000 tr/min pendant 20 min ;
- Ajouter 5 ml de SDS (0.1 %) ;
- Incuber 24h à 37 °C avec agitation ;
- Centrifuger la suspension lysée à 9000 tr/min pendant 15 min, deux fois ;
- Dissoudre dans l'hypochlorite de sodium (30 %) ;
- Incuber à 30 °C pendant 3 min ;
- Centrifuger à 9000 tr/min pendant 15 min ;
- Jeter le surnageant et laver le culot avec de l'eau distillée puis un mélange acétone/ alcool (1 :1) ;
- Bien vortexer ;
- dissoudre dans le chloroforme à chaud jusqu'à évaporation.

Des résultats d'extraction de PHA, à partir de cultures réalisées en grand volume (1,5L), comprenant de 2,1 à 2,5 g de biomasse, sont consignés dans le tableau 1 suivant.

Tableau 1

| **Substrat utilisé** | **Quantité de PHA extrait (mg)** |
|---|---|
| Huile de colza **(6)** | 90 |
| Huile de colza + Glucose **(3)** | 140 |
| Glycérol + Glucose **(2)** | 40 |
| Glucose **(1)** | 120 |

**Exemple 3 : Production de PHA sans stérilisation du milieu de culture**

La croissance de la souche S3S1 a pu être obtenue dans des milieux comprenant 150 ou 200 g/L de NaCl, alors que les dits milieux n'ont pas été autoclavés.

**Exemple 4 : Structure des PHA**

Le PHA obtenu à partir du substrat **3** (Huile de colza + Glucose, exemple Ibis) a été analysé par RMN, DSC et GPC.

• **Matériel et méthodes**

**Analyse par RMN**

Les spectres RMN $^1$H et $^{13}$C sont réalisés dans du $CDCl_3$ (appareil Bruker 400MHz).

**Analyse par DSC**

Les propriétés thermiques sont déterminées par DSC (Diamon DSC - Perkin Elmer). La température de fusion est déterminée au 1$^{er}$ passage et la température de transition vitreuse au 2$^{ème}$ passage.
Le programme de température utilisé est le suivant:

1) de 20°C à -50°C à 40°C/min ;
2) 3 min à -50°C ;
3) 1er passage : de -50°C à 200°C à 20°C/min ;
4) de 180°C à -50°C à 200°C/min ;
5) 3 min à -50°C ;
6) 2eme passage : -50°C à 200°C à 20°C/min.

**Analyse par GPC**

Les échantillons sont dissous dans le chloroforme (5 mg/mL) avant analyse. Deux modes de détection ont été utilisés : le réfractomètre différentiel (RI) et la diffusion de lumière (ddl). Les masses molaires déterminées par RI sont données en équivalent polystyrène.
Les appareils utilisés sont les suivants : la pompe Shimadzu LC-20AD est équipée de 2 colonnes PL Gel Polymer Laboratories 5μm mixte C et de deux détecteurs : un réfractomètre différentiel Wyatt Optilab Rex et un détecteur diffusion de la lumière Wyatt Dawn Heleos 8. Les polymères sont analysés par diffusion de lumière en utilisant un dn/dc de 0,034, valeur caractéristique des PHAs à courtes chaînes latérales dans le chloroforme.

• **Résultats**

**Analyse par DSC**

Les températures de fusion (Tf) et les enthalpies de fusion sont déterminées au 1$^{er}$ passage, et les températures de transition vitreuse (Tg) au 2$^{ème}$ passage. La Tg est mesurée au point d'inflexion, la Tf est mesurée au sommet du pic.
Le pic exothermique est dû au phénomène de cristallisation et il apparaît au cours du 2$^{ème}$ passage.
Les résultats sont rassemblés dans le tableau 2.

Tableau 2

| **Mesures réalisées** | **Résultats** |
|---|---|
| Tg (°C) | +5 |
| Tf(°C) | 157 |
| ΔHF (J/g) | 70 |

**Détermination des masses molaires par SEC**

Les résultats concernant les masses molaires moyennes en nombre Mn, les masses molaires moyennes en poids Mw et l'indice de polymolécularité Ip (parfois nommé indice de polydispersité) sont rassemblés dans le tableau 3.

Tableau 3

| **Mesures réalisées** | Mn(RI) | Mw(RI) | Ip(RI) | Mn(ddl) | Mw(ddl) | Ip(ddl) |
|---|---|---|---|---|---|---|
| **Résultats** | 642 000 | 1 097 000 | 1.7 | 597 400 | 732 900 | 1.2 |

**Détermination de la structure chimique par RMN.**

Les spectres RMN $^1H$ et $^{13}C$ ainsi que les attributions sont respectivement présentés aux **figures 2** et **3.**

• **Conclusion**

En RMN du proton, les pics à 1.2, 2.5 et 5.2 ppm sont caractéristiques respectivement des groupes $CH_3$, $CH_2$ et CH du poly(3-hydroxybutyrate), PHB (figure 2). Ces groupements sont également clairement identifiés par RMN $^{13}C$ (19.6, 40.9 et 67.7 ppm). Le pic à 169 ppm est également caractéristique de la fonction ester **(figure 3).**

L'échantillon est majoritairement constitué de PHB. Cependant la RMN indique la présence d'autres groupements en plus faible quantité.

En RMN $^1H$, les pics à 0.8, 1.5 et 5.05 sont caractéristiques de la présence de motifs hydroxyvalérate (HV) ($CH_3$, $CH_2$ de la chaîne latérale et CH du squelette macromoléculaire).

En RMN $^{13}C$, ces groupements sont également visibles à 9.5, 27 et 72 ppm.

En faisant les rapports des intégrations des pics situés à 0.8 ppm (PHV) et à 1,2 ppm (PHB), nous pouvons en déduire que la proportion de motif HV est de 4%.

Il s'agit donc d'un polymère P(HB-co-HV) dans des proportions 96/4.

Cette structure chimique est cohérente avec les résultats obtenus en DSC car la température de transition vitreuse Tg est de 5°C et la température de fusion Tf est de 157°C, ce qui est en accord avec les résultats connus pour les PHAs scl.

Il est à noter, à propos du comportement dudit polymère en solution que ladite solution est parfaitement limpide mais très visqueuse. La masse molaire en poids déterminée par ddl est particulièrement élevée (732 900 g/mol) et l'indice de polymolécularité est de 1.7 (tableau 3).

## Revendications

1. Utilisation de la souche hyperhalophile aérobie S3S1 déposée le 21 février 2014 à la CNCM sous le numéro CNCM I-4838
pour la mise en œuvre d'un procédé de dégradation :

    - d'un substrat constitué de ou comprenant un élément choisi parmi les triglycérides, leurs sous-produits, les alcanes, les alcènes, les polyènes et leurs mélanges ;
    - et éventuellement d'un co-substrat carboné,

    contenus dans le milieu de culture de la dite souche hyperhalophile aérobie et la préparation d'un biopolymère de type polyhydroxyalkanoate (PHA), ledit PHA étant de masse moléculaire en poids supérieure à 600 000 g/mol.

2. Procédé de dégradation :

    - d'un substrat constitué de ou comprenant un élément choisi parmi les triglycérides, leurs sous-produits, les alcanes, les alcènes, les polyènes et leurs mélanges ;
    - et éventuellement un co-substrat carboné,

    comprenant :

    - une étape de croissance d'une souche hyperhalophile aérobie S3S1 déposée le 21 février 2014 à la CNCM sous le numéro CNCM I-4838 dans un milieu de culture comprenant :

        ◦ un substrat constitué de ou comprenant un élément choisi parmi les triglycérides, leurs sous-produits, les alcanes, les alcènes, les polyènes et leurs mélanges ;
        ◦ et éventuellement un co-substrat carboné ;

    ledit procédé de dégradation étant mis en œuvre pour la préparation d'un biopolymère de type polyhydroxyalkanoate (PHA), ledit PHA étant de masse moléculaire en poids supérieure à 600 000 g/mol.

3. Procédé de dégradation d'un substrat carboné selon la revendication 2, dans lequel le milieu de culture de ladite souche comprend un substrat choisi parmi le groupe constitué :

- des huiles végétales et animales, en particulier les huiles végétales, plus particulièrement l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, leurs mélanges, et leurs dérivés, en particulier les huiles de friture ;
- des alcanes linéaires, ramifiés ou cycliques, comprenant de 6 à 24 atomes de carbone, en particulier de 10 à 22 atomes de carbone ;
- des alcènes linéaires, ramifiés ou cycliques, comprenant de 6 à 24 atomes de carbone, en particulier de 10 à 22 atomes de carbone ;
- des polyènes linéaires, ramifiés ou cycliques, comprenant de 6 à 24 atomes de carbone, en particulier de 10 à 22 atomes de carbone ;
- des acides gras, en particulier les acides gras saturés et insaturés, plus particulièrement les acides gras mono insaturés, di insaturés et poly insaturés, lesdits acides gras comprenant un acide carboxylique et une chaîne comprenant de 6 à 24 atomes de carbone, en particulier de 10 à 22 atomes de carbone, ainsi que leurs dérivés, en particulier les esters des dits acides gras, plus particulièrement les esters méthyliques et éthyliques des dits acides gras;
- des trigycérides ;
- du glycérol ;
- des co-produits des raffineries d'huile, en particulier d'huile végétale, notamment les pâtes de neutralisation, les condensats de désodorisation, les gommes, les distillats d'acides gras, les graisses d'aéroflottateurs et les boues de station d'épuration,

et leurs mélanges,
contenu dans le milieu de culture de la dite souche hyperhalophile aérobie.

**4.** Procédé de dégradation d'un substrat carboné selon l'une quelconque des revendications 2 à 3, dans lequel le milieu de culture de ladite souche comprend un substrat et un co-substrat carboné, ledit co-substrat étant un sucre, en particulier le glucose.

**5.** Procédé selon l'une quelconque des revendications 2 à 4, dans lequel le milieu de culture comprend au moins un sel, en particulier choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges,
le rapport massique $NaCl/MgCl_2$ étant notamment compris de 15 à 100, et/ou le rapport massique $NaCl/CaCl_2$ étant notamment compris de 20 à 100.

**6.** Procédé selon l'une quelconque des revendications 2 à 5 dans lequel :

- ladite souche est cultivée sous une atmosphère comprenant moins de 21% en volume de dioxygène, en particulier moins de 20%, 19%, 18%, 17%, 16%, 15%, 14%, 13%, 12%, 11%, 10%, 9%, 8%, 7%, 6%, 5%, 4%, 3%, 2% ou 1% en volume de dioxygène, ou
- le rapport carbone sur azote dans le milieu de culture est compris de 1 à 300, ou
- le rapport carbone sur phosphore dans le milieu de culture est compris de 80 à 800.

**7.** Procédé de préparation d'un biopolymère de type PHA de masse moléculaire en poids supérieure à 600 000 g/mol comprenant :

(a) une étape de lyse d'une culture de la souche hyperhalophile aérobie S3S1 déposée le 21 février 2014 à la CNCM sous le numéro CNCM I-4838,
ladite culture étant obtenue après croissance dans un milieu comprenant :

- d'un substrat constitué de ou comprenant un élément choisi parmi les triglycérides, leurs sous-produits, les alcanes, les alcènes, les polyènes et leurs mélanges ;

et éventuellement un co-substrat carboné, ledit co-substrat étant un sucre, en particulier le glucose.

**8.** Procédé selon la revendication 7, dans lequel l'étape de lyse (a) comprend :

- la mise en contact de la dite culture de la dite souche, optionnellement sous forme lyophilisée, avec un détergent pour obtenir un lysat cellulaire, ou

- la sonication de la dite culture de la dite souche pour obtenir un lysat cellulaire, ou
- la mise en contact de la dite culture de la dite souche, optionnellement sous forme lyophilisée, avec un détergent, puis la sonication du mélange ainsi obtenu pour obtenir un lysat cellulaire.

**9.** Procédé selon l'une quelconque des revendications 7 à 8 comprenant une étape (b) d'extraction du PHA à partir du lysat cellulaire,
ladite extraction du PHA est en particulier mise en œuvre par précipitation en ajoutant de l'hypochlorite de sodium, notamment de l'hypochlorite de sodium à au moins 25%, en particulier 30%, de chlore actif, dans le culot cellulaire obtenu par centrifugation du lysat cellulaire, en particulier dans un rapport 1:1 volume à volume,
pour obtenir le dit biopolymère de type PHA et une fraction cellulaire.

**10.** Procédé selon l'une quelconque des revendications 7 à 9 comprenant (c) une étape de purification du PHA,
ladite étape de purification du PHA étant en particulier mise en œuvre par ajout d'un solvant organique, notamment le chloroforme, en particulier à une température comprise de 20°C à 80°C, plus particulièrement à une température de 45°C à 65°C, au dit biopolymère de type PHA et à ladite fraction cellulaire, pour obtenir une solution de PHA dans ledit solvant organique, ladite solution étant dépourvue de la fraction cellulaire.

**11.** Procédé de préparation d'un biopolymère de type PHA selon l'une quelconque des revendications 7 à 10 dans lequel la dite souche est cultivée sur un milieu comprenant un substrat choisi parmi le groupe constitué :

- des huiles végétales et animales, en particulier les huiles végétales, plus particulièrement l'huile de colza, l'huile de tournesol, l'huile de maïs, l'huile de lin, l'huile d'olive, l'huile de ricin, l'huile de soja, l'huile de palme, l'huile palmiste, l'huile de coco, l'huile de graine de coton, l'huile d'arachide, leurs mélanges, et leurs dérivés, en particulier les huiles de friture ;
- des alcanes linéaires, ramifiés ou cycliques, comprenant de 6 à 24 atomes de carbone, en particulier de 10 à 22 atomes de carbone ;
- des alcènes linéaires, ramifiés ou cycliques, comprenant de 6 à 24 atomes de carbone, en particulier de 10 à 22 atomes de carbone ;
- des polyènes linéaires, ramifiés ou cycliques, comprenant de 6 à 24 atomes de carbone, en particulier de 10 à 22 atomes de carbone ;
- des acides gras, en particulier les acides gras saturés et insaturés, plus particulièrement les acides gras mono insaturés, di insaturés et poly insaturés, lesdits acides gras comprenant un acide carboxylique et une chaîne comprenant de 6 à 24 atomes de carbone, en particulier de 10 à 22 atomes de carbone, ainsi que leurs dérivés, en particulier les esters des dits acides gras, plus particulièrement les esters méthyliques et éthyliques des dits acides gras ;
- des trigycérides ;
- du glycérol ;
- des co-produits des raffineries d'huile, en particulier d'huile végétale, notamment les pâtes de neutralisation, les condensats de désodorisation, les gommes, les distillats d'acides gras, les graisses d'aéroflottateurs et les boues de station d'épuration.

**12.** Procédé selon l'un quelconque des revendications 7 à 11 dans lequel la dite souche est cultivée dans un milieu comprenant au moins un sel, en particulier choisi parmi NaCl, KCl, $CaCl_2$, $MgCl_2$, $MgSO_4$, leurs hydrates, en particulier $CaCl_2.2H_2O$ et $MgCl_2.6H_2O$, et leurs mélanges,
le rapport massique $NaCl/MgCl_2$ étant notamment compris de 15 à 100, et/ou le rapport massique $NaCl/CaCl_2$ étant notamment compris de 20 à 100.

**13.** Souche hyperhalophile aérobie S3S1 déposée le 21 février 2014 à la CNCM sous le numéro CNCM 1-4838.

**Patentansprüche**

**1.** Verwendung des aeroben hyperhalophilen Stamms S3S1, der am 21. Februar 2014 beim CNCM unter der Nummer CNCM I-4838 hinterlegt wurde,
zur Durchführung eines Verfahren zum Abbau:

- eines Substrats, das aus einem Element, ausgewählt aus Triglyceriden, Nebenprodukten davon, Alkanen, Alkenen, Polyenen und Mischungen davon, besteht oder dieses umfasst;

- und gegebenenfalls einem kohlenstoffhaltigen Co-Substrat,

welche in dem Kulturmedium des aeroben hyperhalophilen Stamms enthalten sind, und zur Herstellung eines Biopolymers vom Typ Polyhydroxyalkanoat (PHA), wobei das PHA ein Molekulargewicht von mehr als 600.000 g/mol aufweist.

**2.** Verfahren zum Abbau:

- eines Substrats, das aus einem Element, ausgewählt aus Triglyceriden, Nebenprodukten davon, Alkanen, Alkenen, Polyenen und Mischungen davon, besteht oder dieses umfasst;
- und gegebenenfalls einem kohlenstoffhaltigen Co-Substrat,

umfassend:

- einen Schritt zum Kultivieren eines aeroben hyperhalophilen S3S1-Stamms, der am 21. Februar 2014 beim CNCM unter der Nummer CNCM I-4838 hinterlegt wurde, in einem Kulturmedium, das Folgendes umfasst:

  ◦ ein Substrat, das aus einem Element, das aus Triglyceriden, Nebenprodukten davon, Alkanen, Alkenen, Polyenen und Mischungen davon, besteht oder dieses umfasst;
  ◦ und gegebenenfalls einem kohlenstoffhaltigen Co-Substrat;

wobei das Abbauverfahren zur Herstellung eines Biopolymers vom Typ Polyhydroxyalkanoat (PHA) durchgeführt wird, wobei das PHA ein Molekulargewicht von mehr als 600.000 g/mol aufweist.

**3.** Verfahren zum Abbau eines kohlenstoffhaltigen Substrats nach Anspruch 2, wobei das Kulturmedium des Stamms ein Substrat umfasst, ausgewählt aus der Gruppe bestehend aus:

- pflanzlichen und tierischen Öle, insbesondere pflanzlichen Ölen, insbesondere Rapsöl, Sonnenblumenöl, Maisöl, Leinöl, Olivenöl, Rizinusöl, Sojaöl, Palmöl, Palmkernöl, Kokosöl, Baumwollsaatöl, Erdnussöl, Mischungen davon, sowie Derivaten davon, insbesondere Frittieröl;
- linearen, verzweigten oder cyclischen Alkanen, umfassend 6 bis 24 Kohlenstoffatome, insbesondere 10 bis 22 Kohlenstoffatome;
- linearen, verzweigten oder cyclischen Alkanen, umfassend 6 bis 24 Kohlenstoffatome, insbesondere 10 bis 22 Kohlenstoffatome;
- linearen, verzweigten oder cyclischen Polyenen, umfassend 6 bis 24 Kohlenstoffatome, insbesondere 10 bis 22 Kohlenstoffatome;
- Fettsäuren, insbesondere gesättigten und ungesättigten Fettsäuren, insbesondere ein-, zwei- und mehrfach ungesättigten Fettsäuren, wobei die Fettsäuren eine Carbonsäure und eine Kette mit 6 bis 24 Kohlenstoffatomen, insbesondere 10 bis 22 Kohlenstoffatomen umfassen, sowie Derivaten davon insbesondere Fettsäureestern, insbesondere Fettsäuremethylestern und Fettsäureethylestern;
- Trigyceriden;
- Glycerin;
- Nebenprodukten von Ölraffinerien, insbesondere von Pflanzenölraffinerien, insbesondere Neutralisationspasten, Desodorierungskondensaten, Gummen, Fettsäuredestillaten, Fetten aus der Entspannungsflotation und Klärschlämmen,

und Mischungen davon,
die im Kulturmedium des aeroben hyperhalophilen Stamms enthalten sind.

**4.** Verfahren zum Abbau eines kohlenstoffhaltigen Substrats nach einem der Ansprüche 2 bis 3, wobei das Kulturmedium des Stamms ein Substrat und ein kohlenstoffhaltiges Co-Substrat umfasst, wobei das Co-Substrat ein Zucker, insbesondere Glucose, ist.

**5.** Verfahren nach einem der Ansprüche 2 bis 4, wobei das Kulturmedium mindestens ein Salz umfasst, das insbesondere ausgewählt ist aus NaCl, KCl, $CaCl_2$, $MgCl_2$, $MgSO_4$, Hydraten davon, insbesondere $CaCl_2.2H_2O$ und $MgCl_2$. $6H_2O$, und Mischungen davon, wobei das $NaCl/MgCl_2$-Gewichtsverhältnis insbesondere zwischen 15 und 100, und/oder das $NaCl/CaCl_2$-Gewichtverhältnis insbesondere zwischen 20 und 100 liegt.

**6.** Verfahren nach einem der Ansprüche 2 bis 5, wobei:

- der Stamm unter einer Atmosphäre kultiviert wird, die weniger als 21 Vol.-% Disauerstoff umfasst, insbesondere weniger als 20 Vol.-%, 19 Vol.-%, 18 Vol.-%, 17 Vol.-%, 16 Vol.-%, 15 Vol.-%, 14 Vol.-%, 13 Vol.-%, 12 Vol.-%, 11 Vol.-%, 10 Vol.-%, 9 Vol.-%, 8 Vol.-%, 7 Vol.-%, 6 Vol.-%, 5 Vol.-%, 4 Vol.-%, 3 Vol.-%, 2 Vol.-% oder 1 Vol.-% Disauerstoff umfasst, oder
- das Kohlenstoff-Stickstoff-Verhältnis in dem Kulturmedium zwischen 1 und 300 liegt, oder
- das Kohlenstoff-Phosphor-Verhältnis in dem Kulturmedium zwischen 80 und 800 liegt.

**7.** Verfahren zur Herstellung eines Biopolymers vom Typ PHA mit einem Molekulargewicht von mehr als 600.000 g/mol, umfassend:

(a) einen Schritt zur Lyse einer Kultur des aeroben hyperhalophilen Stamms S3S1, der am 21. Februar 2014 beim CNCM unter der Nummer CNCM I-4838 hinterlegt wurde,

wobei die Kultur nach dem Kultivieren in einem Medium erhalten wird, umfassend:

- ein Substrat, das aus einem Element, ausgewählt aus Triglyceriden, Nebenprodukten davon, Alkanen, Alkenen, Polyenen und Mischungen davon, besteht oder dieses umfasst;

und gegebenenfalls einem kohlenstoffhaltigen Co-Substrat, wobei das Co-Substrat ein Zucker, insbesondere Glukose, ist.

**8.** Verfahren nach Anspruch 7, wobei der Lyseschritt (a) umfasst:

- das Inkontaktbringen der Kultur des Stamms, gegebenenfalls in lyophilisierter Form, mit einem Detergens, um ein Zelllysat zu erhalten, oder
- das Beschallen der Kultur des Stamms, um ein Zelllysat zu erhalten, oder
- das Inkontaktbringen der Kultur des Stamms, gegebenenfalls in lyophilisierter Form, mit einem Detergens, dann das Beschallen der so erhaltenen Mischung, um ein Zelllysat zu erhalten.

**9.** Verfahren nach einem der Ansprüche 7 bis 8, umfassend einen Schritt (b) zum Extrahieren von PHA aus dem Zelllysat,
wobei die PHA-Extraktion insbesondere durch Ausfällen durchgeführt wird, indem Natriumhypochlorit, insbesondere mindestens 25 % Natriumhypochlorit, insbesondere 30 % aktives Chlor, zu dem durch Zentrifugation des Zelllysats erhaltenen Zellpellets, insbesondere in einem Verhältnis von 1:1 (Vol./Vol.) gegeben wird,
um das Biopolymer vom Typ PHA und eine Zellfraktion zu erhalten.

**10.** Verfahren nach einem der Ansprüche 7 bis 9, umfassend (c) einen Schritt zum Aufreinigen von PHA,
wobei der Schritt zum Aufreinigen von PHA insbesondere durch Zugeben eines organischen Lösungsmittels, insbesondere Chloroform, insbesondere bei einer Temperatur zwischen 20 °C und 80 °C, insbesondere bei einer Temperatur zwischen 45 °C und 65 °C, zu dem PHA-Biopolymer und zu der Zellfraktion durchgeführt wird, um eine Lösung von PHA in dem organischen Lösungsmittel zu erhalten, wobei die Lösung frei von der Zellfraktion ist.

**11.** Verfahren zur Herstellung eines Biopolymers vom Typ PHA nach einem der Ansprüche 7 bis 10, wobei der Stamm auf einem Medium kultiviert wird, das ein Substrat umfasst, das ausgewählt ist aus der Gruppe bestehend aus:

- pflanzlichen und tierischen Ölen, insbesondere pflanzlichen Ölen, insbesondere Rapsöl, Sonnenblumenöl, Maisöl, Leinöl, Olivenöl, Rizinusöl, Sojaöl, Palmöl, Palmkernöl, Kokosöl, Baumwollsaatöl, Erdnussöl, Mischungen davon, sowie Derivaten davon, insbesondere Frittierölen;
- linearen, verzweigten oder cyclischen Alkanen, umfassend 6 bis 24 Kohlenstoffatome, insbesondere 10 bis 22 Kohlenstoffatome;
- linearen, verzweigten oder cyclischen Alkenen, umfassend 6 bis 24 Kohlenstoffatome, insbesondere 10 bis 22 Kohlenstoffatome;
- linearen, verzweigten oder cyclischen Polyenen, umfassend 6 bis 24 Kohlenstoffatome, insbesondere 10 bis 22 Kohlenstoffatome;
- Fettsäuren, insbesondere gesättigten und ungesättigten Fettsäuren, insbesondere ein-, zwei- und mehrfach ungesättigten Fettsäuren, wobei die Fettsäuren eine Carbonsäure und eine Kette mit 6 bis 24 Kohlenstoffatomen,

insbesondere 10 bis 22 Kohlenstoffatomen umfassen, sowie Derivaten davon, insbesondere Fettsäureestern, insbesondere Fettsäuremethyl- und Fettsäureethylestern;
- Trigyceriden;
- Glycerin;
- Nebenprodukten von Ölraffinerien, insbesondere von Pflanzenölraffinierien, insbesondere Neutralisationspasten, Desodorierungskondensaten, Gummen, Fettsäuredestillaten, Fetten aus der Entspannungsflotation und Klärschlämmen.

**12.** Verfahren nach einem der Ansprüche 7 bis 11, wobei der Stamm in einem Medium kultiviert wird, das mindestens ein Salz umfasst, insbesondere ausgewählt aus NaCl, KCl, $CaCl_2$, $MgCl_2$, $MgSO_4$, Hydrate davon, insbesondere $CaCl_2.2H_2O$ und $MgCl_2.6H_2O$, und Mischungen davon,
wobei das NaCl/$MgCl_2$-Gewichtsverhältnis insbesondere zwischen 15 und 100 liegt und/oder das NaCl/$CaCl_2$-Gewichtsverhältnis insbesondere zwischen 20 und 100 liegt.

**13.** Aerober hyperhalophiler Stamm S3S1, der am 21. Februar 2014 beim CNCM unter der Nummer CNCM I-4838 hinterlegt wurde.

## Claims

**1.** Use of the aerobic hyperhalophilic strain S3S1 deposited on 21 February 2014 at the CNCM under the number CNCM 1-4838
for implementing a process for the degradation of:

- a substrate constituted by or comprising an element selected from triglycerides, by-products thereof, alkanes, alkenes, polyenes and mixtures thereof;
- and optionally of a carbon-containing co-substrate,

contained in the culture medium of said aerobic hyperhalophilic strain and for preparing a biopolymer of the polyhydroxyalkanoate (PHA) type, said PHA having a molecular weight above 600 000 g/mol.

**2.** Process for the degradation of:

- a substrate constituted by or comprising an element selected from triglycerides, by-products thereof, alkanes, alkenes, polyenes and mixtures thereof;
- and optionally of a carbon-containing co-substrate,

comprising:

- a growth step for an aerobic hyperhalophilic strain S3S1 deposited on 21 February 2014 at the CNCM under the number CNCM 1-4838 in a culture medium comprising:

  ◦ a substrate constituted by or comprising an element selected from triglycerides, by-products thereof, alkanes, alkenes, polyenes and mixtures thereof;
  ◦ and optionally a carbon-containing co-substrate.

said process of degradation being implemented for preparing a biopolymer of the polyhydroxyalkanoate (PHA) type, said PHA having a molecular weight above 600 000 g/mol.

**3.** Process for the degradation of a carbon-containing substrate according to claim 2, in which the culture medium of said strain comprises a substrate selected from the group constituted by:

- vegetable and animal oils, in particular vegetable oils, more particularly rapeseed oil, sunflower oil, maize oil, linseed oil, olive oil, castor oil, soya oil, palm oil, palm kernel oil, coconut oil, cottonseed oil, peanut oil, mixtures thereof, and derivatives thereof, in particular frying oils;
- linear, branched or cyclic alkanes, comprising from 6 to 24 carbon atoms, in particular from 10 to 22 carbon atoms;
- linear, branched or cyclic alkenes, comprising from 6 to 24 carbon atoms, in particular from 10 to 22 carbon

atoms;
- linear, branched or cyclic polyenes, comprising from 6 to 24 carbon atoms, in particular from 10 to 22 carbon atoms;
- fatty acids, in particular saturated and unsaturated fatty acids, more particularly monounsaturated, diunsaturated and polyunsaturated fatty acids, said fatty acids comprising a carboxylic acid and a chain comprising from 6 to 24 carbon atoms, in particular from 10 to 22 carbon atoms, and derivatives thereof, in particular the esters of said fatty acids, more particularly the methyl and ethyl esters of said fatty acids;
- triglycerides;
- glycerol;
- co-products from oil refineries, in particular of vegetable oil, in particular neutralization pastes, deodorization condensates, gums, fatty acid distillates, fats from air flotation units and sludges from treatment plants,

and mixtures thereof,
contained in the culture medium of said aerobic hyperhalophilic strain.

**4.** Process for the degradation of a carbon-containing substrate according to any one of claims 2 or 3, in which the culture medium of said strain comprises a substrate and a carbon-containing co-substrate, said co-substrate being a sugar, in particular glucose.

**5.** Process according to any one of claims 2 to 4, in which the culture medium comprises at least one salt, in particular selected from NaCl, KCl, $CaCl_2$, $MgCl_2$, $MgSO_4$, hydrates thereof, in particular $CaCl_2.2H_2O$ and $MgCl_2.6H_2O$, and mixtures thereof,
the $NaCl/MgCl_2$ weight ratio in particular being comprised from 15 to 100, and/or
the $NaCl/CaCl_2$ weight ratio in particular being comprised from 20 to 100.

**6.** Process according to any one of claims 2 to 5, in which:

- said strain is cultured under an atmosphere comprising less than 21% by volume of dioxygen, in particular less than 20%, 19%, 18%, 17%, 16%, 15%, 14%, 13%, 12%, 11%, 10%, 9%, 8%, 7%, 6%, 5%, 4%, 3%, 2% or 1% by volume of dioxygen, or
- the ratio of carbon to nitrogen in the culture medium is comprised from 1 to 300, or
- the ratio of carbon to phosphorus in the culture medium is comprised from 80 to 800.

**7.** Process for the preparation of a biopolymer of the PHA type having a molecular weight above 600 000 g/mol comprising:

(a) a step of lysis of a culture of the aerobic hyperhalophilic strain S3S1 deposited on 21 February 2014 at the CNCM under the number CNCM 1-4838,
said culture being obtained after growth in a medium comprising:

- a substrate constituted by or comprising an element selected from triglycerides, by-products thereof, alkanes, alkenes, polyenes and mixtures thereof;

and optionally a carbon-containing co-substrate, said co-substrate being a sugar, in particular glucose.

**8.** Process according to claim 7, in which the lysis step (a) comprises:

- bringing said culture of said strain, optionally in the lyophilized form, into contact with a detergent in order to obtain a cellular lysate, or
- sonication of said culture of said strain in order to obtain a cellular lysate, or
- bringing said culture of said strain, optionally in the lyophilized form, into contact with a detergent, then sonication of the mixture thus obtained, in order to obtain a cellular lysate.

**9.** Process according to any one of claims 7 or 8 comprising a step (b) of extraction of the PHA from the cellular lysate, said extraction of the PHA is in particular carried out by precipitation by adding sodium hypochlorite, in particular sodium hypochlorite with at least 25%, in particular 30%, of active chlorine, to the cellular pellet obtained by centrifugation of the cellular lysate, in particular in a ratio of 1:1 volume by volume,
in order to obtain said biopolymer of the PHA type and a cellular fraction.

**10.** Process according to any one of claims 7 to 9 comprising a step (c) of purification of the PHA, said step of purification of the PHA being carried out in particular by adding an organic solvent, in particular chloroform, in particular at a temperature comprised from 20°C to 80°C, more particularly at a temperature from 45°C to 65°C, to said biopolymer of the PHA type and to said cellular fraction, in order to obtain a solution of PHA in said organic solvent, said solution being devoid of the cellular fraction.

**11.** Process for the preparation of a biopolymer of the PHA type according to any one of claims 7 to 10, in which said strain is cultured on a medium comprising a substrate selected from the group constituted by:

- vegetable and animal oils, in particular vegetable oils, more particularly rapeseed oil, sunflower oil, maize oil, linseed oil, olive oil, castor oil, soya oil, palm oil, palm kernel oil, coconut oil, cottonseed oil, peanut oil, mixtures thereof, and derivatives thereof, in particular frying oils;
- linear, branched or cyclic alkanes, comprising from 6 to 24 carbon atoms, in particular from 10 to 22 carbon atoms;
- linear, branched or cyclic alkenes, comprising from 6 to 24 carbon atoms, in particular from 10 to 22 carbon atoms;
- linear, branched or cyclic polyenes, comprising from 6 to 24 carbon atoms, in particular from 10 to 22 carbon atoms;
- fatty acids, in particular saturated and unsaturated fatty acids, more particularly monounsaturated, diunsaturated and polyunsaturated fatty acids, said fatty acids comprising a carboxylic acid and a chain comprising from 6 to 24 carbon atoms, in particular from 10 to 22 carbon atoms, and derivatives thereof, in particular the esters of said fatty acids, more particularly the methyl and ethyl esters of said fatty acids;
- triglycerides;
- glycerol;
- co-products from oil refineries, in particular of vegetable oil, in particular neutralization pastes, deodorization condensates, gums, fatty acid distillates, fats from air flotation units and sludges from treatment plants.

**12.** Process according to any one of claims 7 to 11, in which said strain is cultured in a medium comprising at least one salt, in particular selected from NaCl, KCl, $CaCl_2$, $MgCl_2$, $MgSO_4$, hydrates thereof, in particular $CaCl_2.2H_2O$ and $MgCl_2.6H_2O$, and mixtures thereof, the NaCl/$MgCl_2$ weight ratio in particular being comprised from 15 to 100, and/or the NaCl/$CaCl_2$ weight ratio in particular being comprised from 20 to 100.

**13.** Aerobic hyperhalophilic strain S3S1 deposited on 21 February 2014 at the CNCM under the number CNCM I-4838.

Figure 1

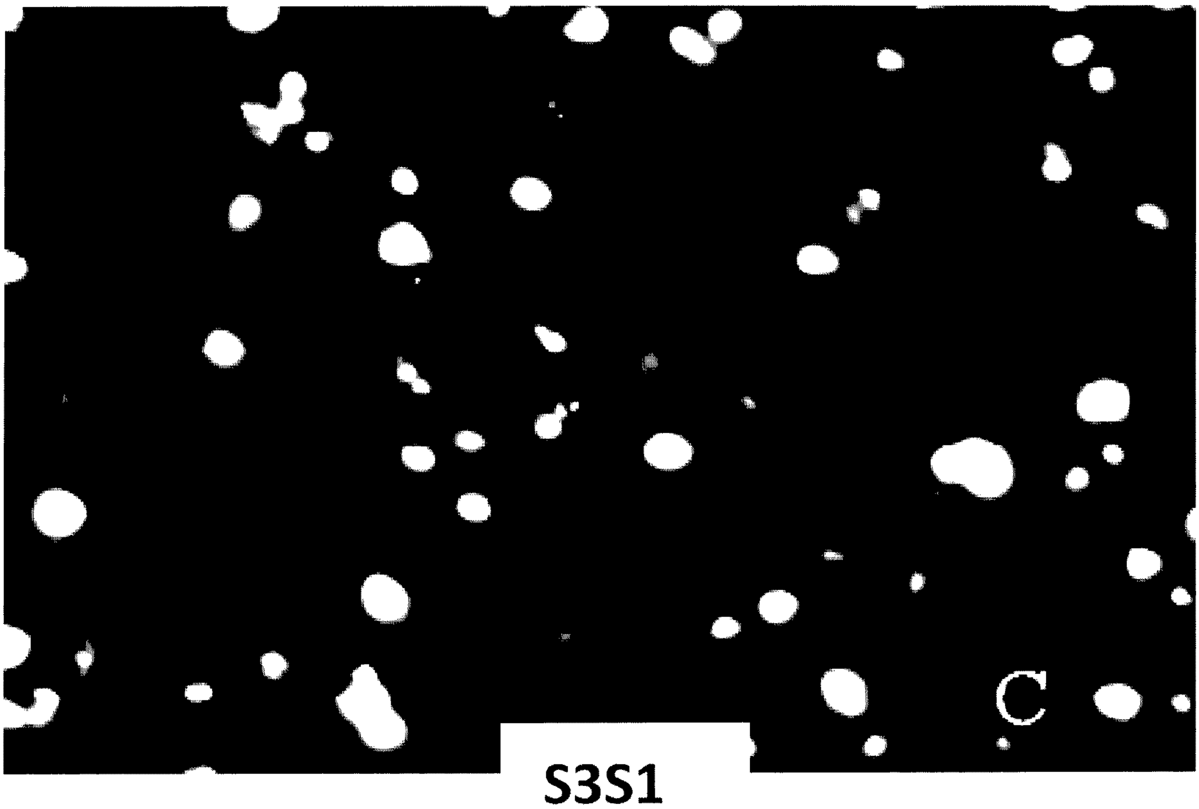

Figure 2

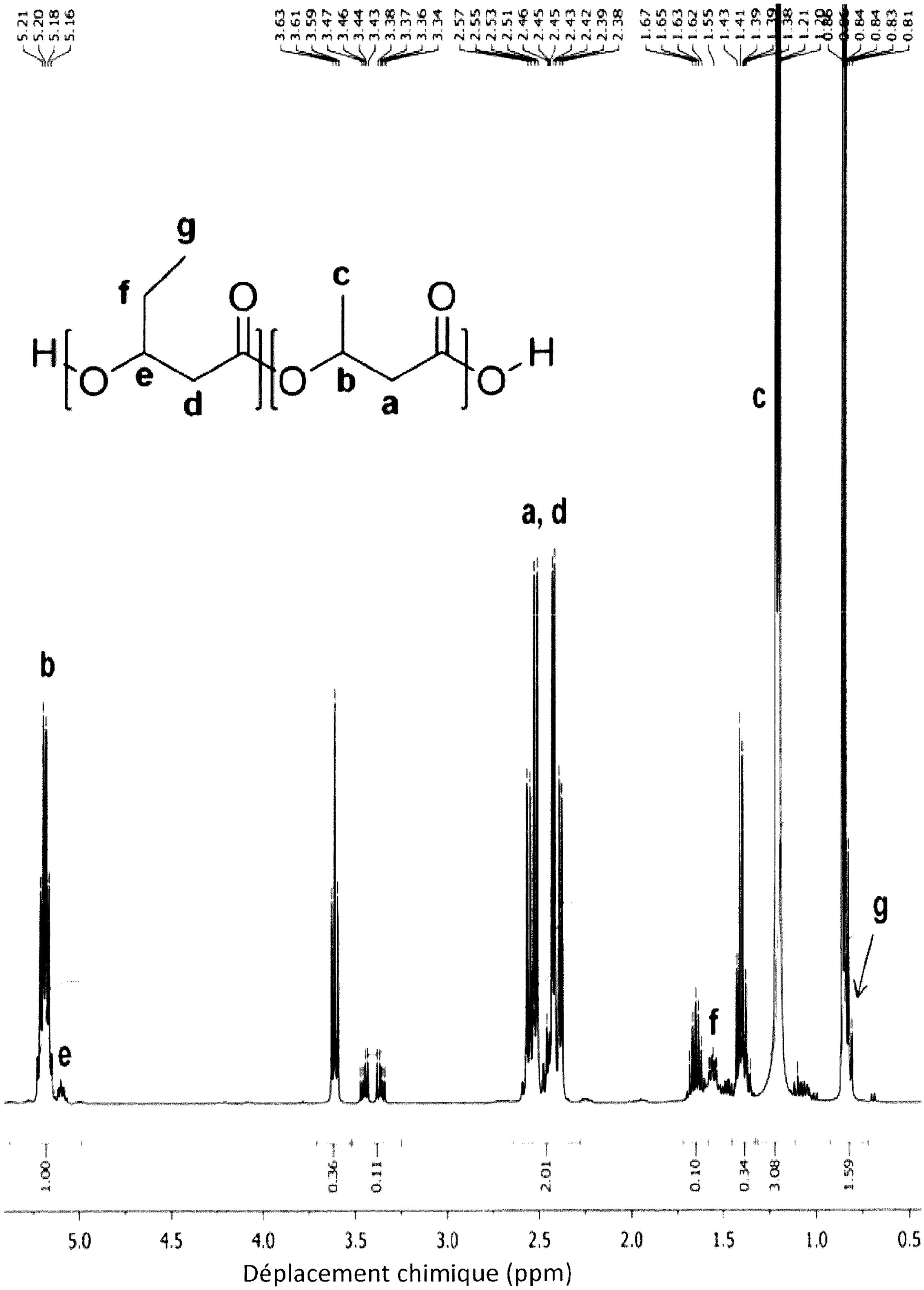

Figure 3

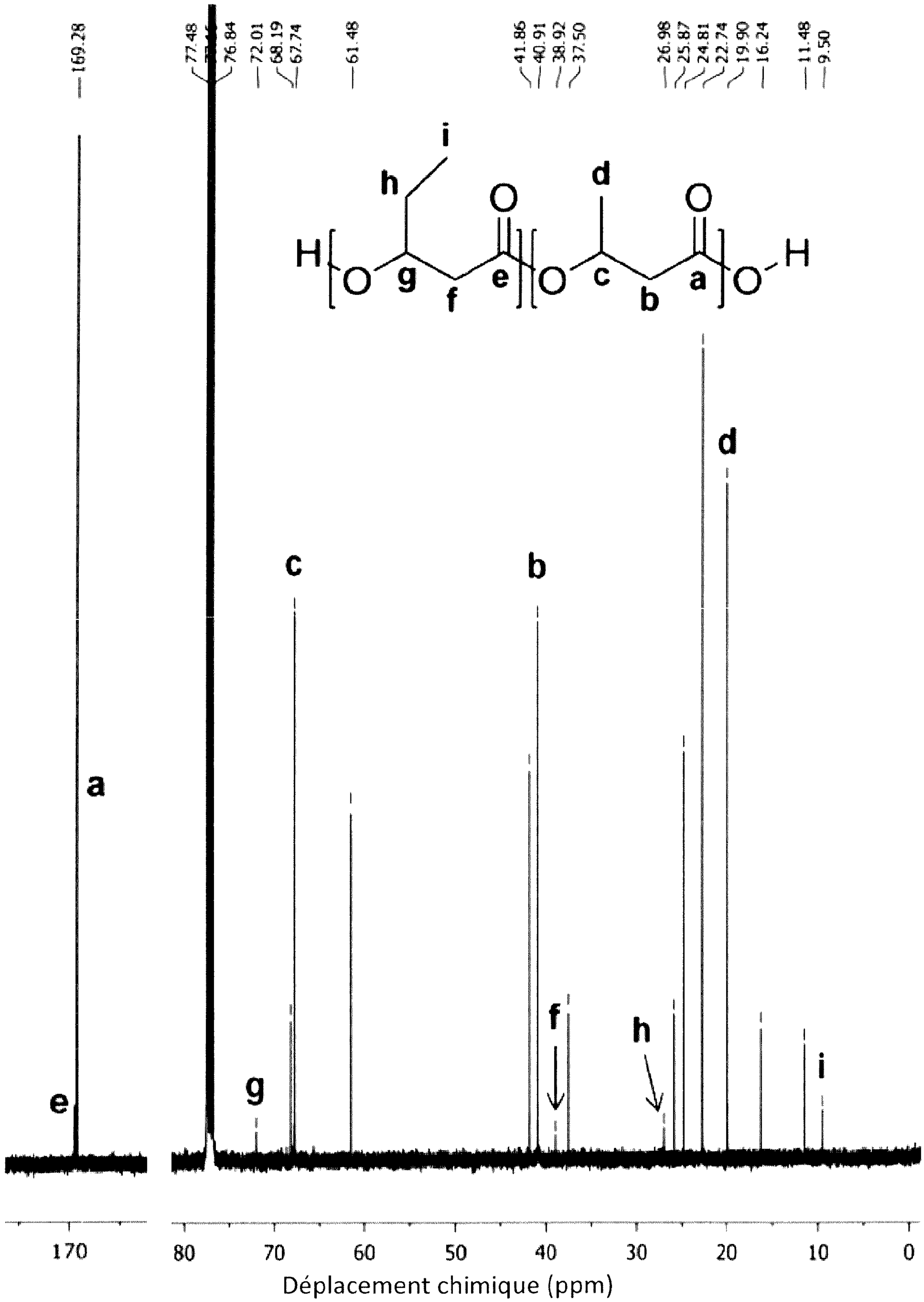
169.28
77.48
76.84
72.01
68.19
67.74
61.48
41.86
40.91
38.92
37.50
26.98
25.87
24.81
22.74
19.90
16.24
11.48
9.50
170
80
70
60
50
40
30
20
10
0
Déplacement chimique (ppm)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **LILLO et al.** *Appl. Environ. Microb.,* 1990, vol. 56, 2517-2521
- **HERMANN-KRAUSS et al.** *Archae,* 2013, vol. 50 (10), 1305-1310
- **DE LEY, J. ; CATTOIR, H. ; REYNAERTS, A.** The quantitative measurement of DNA hybridization from renaturation rates. *Eur JBiochem,* 1970, vol. 12, 133-142
- **HUSS, V. A. R. ; FESTL, H. ; SCHLEIFER, K. H.** Studies on the spectrophotometric détermination of DNA hybridization from renaturation rates. *Syst Appl Microbiol,* 1983, vol. 4, 184-192